(19) Europäisches Patentamt
European Patent Office
Office européen des brevets

(11) EP 1 311 508 B1

(12) EUROPÄISCHE PATENTSCHRIFT

(45) Veröffentlichungstag und Bekanntmachung des Hinweises auf die Patenterteilung:
**17.11.2004 Patentblatt 2004/47**

(21) Anmeldenummer: **01978258.0**

(22) Anmeldetag: **21.07.2001**

(51) Int Cl.$^7$: **C07D 471/04**

(86) Internationale Anmeldenummer:
**PCT/EP2001/008456**

(87) Internationale Veröffentlichungsnummer:
**WO 2002/014313 (21.02.2002 Gazette 2002/08)**

(54) **NEUE BETA-AMYLOID INHIBITOREN, VERFAHREN ZU DEREN HERSTELLUNG UND DEREN VERWENDUNG ALS ARZNEIMITTEL**

NOVEL BETA-AMYLOID INHIBITORS, METHOD FOR PRODUCING THE SAME AND THE USE THEREOF AS MEDICAMENTS

NOUVEAUX INHIBITEURS DE BETA-AMYLOIDE, LEURS PROCEDES DE PRODUCTION ET LEUR UTILISATION COMME MEDICAMENTS

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **16.08.2000 DE 10040016**

(43) Veröffentlichungstag der Anmeldung:
**21.05.2003 Patentblatt 2003/21**

(73) Patentinhaber: **Boehringer Ingelheim Pharma GmbH & Co.KG**
**55218 Ingelheim am Rhein (DE)**

(72) Erfinder:
- **FUCHS, Klaus**
  **55435 Gau-Algesheim (DE)**
- **ROMIG, Helmut,Michael**
  **88400 Biberach an der Riss (DE)**
- **MENDLA, Klaus**
  **55218 Ingelheim am Rhein (DE)**
- **BRIEM, Hans**
  **28279 Bremen (DE)**
- **FECHTELER, Katja**
  **65197 Wiesbaden (DE)**

(56) Entgegenhaltungen:
**EP-A- 0 092 458** **US-A- 6 030 984**

- **TRAPANI G ET AL: "Synthesis and Binding Affinity of 2-Phenylimidazo[1,2-alpha]pyridine Derivatives for both Central and Peripheral Benzodiazepine Receptors. A New Series of High-Affinity and Selective Ligands for the Peripheral Type" JOURNAL OF MEDICINAL CHEMISTRY, AMERICAN CHEMICAL SOCIETY. WASHINGTON, US, Bd. 40, 1997, Seiten 3109-3118, XP002900938 ISSN: 0022-2623**
- **GUPTA S P ET AL: "QUANTITATIVE STRUCTURE-ACTIVITY RELATIONSHIP STUDIES ON SOME NONBENZODIAZEPINE SERIES OF COMPOUNDS ACTING AT THE BENZODIAZEPINE RECEPTOR" BIOORGANIC & MEDICINAL CHEMISTRY, ELSEVIER SCIENCE LTD, GB, Bd. 6, Nr. 11, 1998, Seiten 2213-2218, XP000872293 ISSN: 0968-0896**
- **LOEBER S ET AL: "Azaindole Derivatives with High Affinity for the Dopamine D4 Receptor: Synthesis, Ligand Binding Studies and Comparison of Molecular Electrostatic Potential Maps" BIOORGANIC & MEDICINAL CHEMISTRY LETTERS, OXFORD, GB, Bd. 9, Nr. 1, Januar 1999 (1999-01), Seiten 97-102, XP004154785 ISSN: 0960-894X**
- **LOMBARDINO J G: "Preparation and new reactions of Imidazo[1,2-a]pyridines" JOURNAL OF ORGANIC CHEMISTRY, Nr. 7, 12. Juli 1965 (1965-07-12), Seiten 2403-2407, XP001055720**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 311 508 B1

## Beschreibung

Die vorliegende Erfindung betrifft Verbindungen der allgemeinen Formel (I)

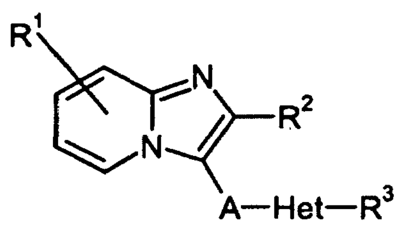

(I)

in der die Reste A, Het, $R^1$, $R^2$ und $R^3$ die in der nachstehenden Beschreibung und in den Ansprüchen genannte Bedeutung haben können, Verfahren zu deren Herstellung sowie die Verwendung von Verbindungen der allgemeinen Formel (I) als Arzneimittel, insbesondere als Arzneimittel mit beta-Amyloid-inhibierender Wirkung.

### Hintergrund der Erfindung

Imidazo[1,2-*a*]pyridine sind aus dem stand der Technik bekannt. Die EP 92 458 offenbart Imidazo[1,2-*a*]pyridine die beispielsweise anxiolytische Wirksamkeit aufweisen. Durch Trapani et al. (J. Med. Chem. 1997, 40, 3109-3118) und Gupta et al. (Bioorg. and Med. Chem. 6 (1998), 2213-2218) werden Imidazo[1,2-*a*]pyridine offenbart, die eine Affinität zu Benzodiazepin-Rezeptoren aufweisen. Löber et al. (Bioorg. and Med. Chem. Lett. 9 (1999) 97 -102) offenbaren Azaindolderivate mit Dopamin D4 Affinität. Synthetische Zugänge zu und Reaktionen von Imidazo [1,2-a]pyridinen werden durch Lombardino beschrieben (J. Org. Chem. 7 (1965) 2403 - 2407) beschrieben.

Aggregation und Präzipitation von Proteinen sind an der Entstehung von verschiedenen neurodegenerativen Erkrankungen wie Alzheimer, Parkinson und Veitstanz (Engl.: "Chorea Huntington") beteiligt. Bei der Alzheimerschen Erkrankung aggregiert das Amyloid-β-peptid (Aβ) und führt zu unlöslichen senilen Plaques, welche einen der pathologischen Marker der Erkrankung darstellen. Aβ entsteht durch proteolytische Spaltung eines Vorläuferproteins, dem Amyloid-Vorläuferprotein (Engl.: "amyloid precursor protein"; Engl.: APP). Man unterscheidet zwei Stoffwechselwege des APP, den nicht amyloidogenen Weg und den amyloidogenen Weg.

Im nicht amyloidogenen Metabolismus des APP spaltet die α-Sekretase innerhalb der Aβ Region des APP und führt damit zur Sekretion des löslichen N-terminalen Bereiches des Proteins (α-APPs) sowie nach erfolgtem γ-Sekretase-Schnitt zur Freisetzung von p3. Dagegen führt der amyloidogene Weg zur Enstehung von Aβ, indem zwei Proteasen den N-Terminus (β-Sekretase) bzw. den C-Terminus (γ-Sekretase) von Aβ generieren.

In vivo ist Aβ im humanen Plasma und der Zerebrospinalflüssigkeit nachzuweisen. Auch in Zellkultur kann sekretiertes Aβ im Zellkulturüberstand in verschiedenen Zelltypen nachgewiesen werden, die APP oder Fragmente davon endogen exprimieren oder überexprimieren.

US 6030984 beschreibt Pyridone, die durch Hemmung der Bildung von β-Amyloid zur Behandlung von Alzheimer Verwendung finden können.

Es ist Aufgabe der vorliegenden Erfindung, Verbindungen bereitzustellen, die geeignet sind, (bevorzugt inhibitorisch) in den Prozeß der Aβ-Entstehung oder Freisetzung aus Zellen eingreifen zu können, oder inhibitorisch die Aβ-Aktivität zu reduzieren. Der vorliegenden Erfindung liegt schließlich die weitere Aufgabe zugrunde, Verbindungen bereitzustellen, die zur Vorbeugung oder Behandlung der Alzheimerschen Erkrankung wirkungsvoll eingesetzt werden können.

### Detaillierte Beschreibung der Erfindung

Die vorstehenden Aufgaben werden durch die nachstehend definierten Verbindungen der allgemeinen Formel (I) gelöst.

Bei den erfindungsgemäßen Verbindungen handelt es sich um Verbindungen der allgemeinen Formel (I) worin

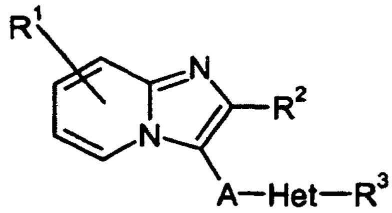

(I)

A $-CH_2-CH_2-$, $-CH_2-CH_2-CH_2-$, $-CH_2-CH_2-CH_2-CH_2-$, $-CH_2-CO$oder -CH=CH-CO- ;
Het Piperidinyl, Piperazinyl oder Dihydrobenzimidazolonyl;
$R^1$ Wasserstoff;
$R^2$ Wasserstoff, $C_1$-$C_4$-Alkyl, $CF_3$ oder ein Phenylrest, der gegebenenfalls durch Halogen, $C_1$-$C_4$-Alkyl oder $C_1$-$C_4$-Alkyloxy substituiert sein kann;
$R^3$ Wasserstoff, $C_1$-$C_4$-Alkyl, HO-$C_1$-$C_4$-Alkyl, $C_2$-$C_4$-Alkenyl, Phenyl oder eine Rest ausgewählt aus der Gruppe bestehend aus Benzyl und Phenylethyl, der gegebenenfalls durch Halogen, $CF_3$, $C_1$-$C_4$-Alkyl oder $C_1$-$C_4$-Alkyloxy substituiert sein kann, oder
ein Heterocyclus ausgewählt aus der Gruppe bestehend aus Morpholin, Piperidin, Piperazin und Dihydrobenzimidazolon, der direkt oder über eine $C_1$-$C_4$-Alkylenbrücke verknüpft sein kann, bedeuten.

Bevorzugt sind Verbindungen der allgemeinen Formel (I), worin

A $-CH_2-CH_2-$, $-CH_2-CH_2-CH_2-$, $-CH_2-CO-$ oder -CH=CH-CO- ;
Het Piperidinyl, Piperazinyl oder Dihydrobenzimidazolonyl;
$R^1$ Wasserstoff;
$R^2$ Wasserstoff, $C_1$-$C_4$-Alkyl, Phenyl, Halogen-substituiertes Phenyl, $C_1$-$C_4$-Alkyloxy-substituiertes Phenyl oder $CF_3$;
$R^3$ $C_1$-$C_4$-Alkyl, HO-$C_1$-$C_4$-Alkyl, $C_2$-$C_4$-Alkenyl, Phenyl, Benzyl, Phenylethyl, Halogen-substituiertes Benzyl oder ein Heterocyclus ausgewählt aus der Gruppe bestehend aus Morpholin und Dihydrobenzimidazolon, der direkt oder über eine $C_1$-$C_4$-Alkylenbrücke verknüpft sein kann, bedeuten.

Besonders bevorzugt sind Verbindungen der allgemeinen Formel (I), worin

A $-CH_2-CH_2-$, $-CH_2-CH_2-CH_2-$, $-CH_2-CO-$ oder -CH=CH-CO- ;
Het Piperidinyl, Piperazinyl oder Dihydrobenzimidazolonyl;
$R^1$ Wasserstoff;
$R^2$ Wasserstoff, Methyl, Phenyl oder 4-Chlorphenyl;
$R^3$ $C_1$-$C_4$-Alkyl, HO-$C_1$-$C_4$-Alkyl, $C_2$-$C_4$-Alkenyl, Phenyl, Benzyl, Halogen-substituiertes Benzyl oder ein Heterocyclus ausgewählt aus Morpholin und Dihydrobenzimidazolon, der direkt oder über eine $C_1$-$C_4$-Alkylenbrücke verknüpft sein kann, bedeuten.

Erfindungsgemäß von besonderer Bedeutung sind Verbindungen der allgemeinen Formel (I), worin

A $-CH_2-CH_2-$, $-CH_2-CH_2-CH_2-$ oder -CH=CH-CO- ;
Het Piperidinyl oder Piperazinyl;
$R^1$ Wasserstoff;
$R^2$ Wasserstoff, Methyl, Phenyl oder 4-Chlorphenyl;
$R^3$ 2-Hydroxyethyl, Phenyl, Benzyl, 4-Chlorbenzyl oder 1,3-Dihydrobenzimidazol-2-on-1-yl, bedeuten.

Bevorzugt sind ferner Verbindungen der allgemeinen Formel (I), worin

A $-CH_2-CH_2-$, $-CH_2-CH_2-CH_2-$ oder -CH=CH-CO- ;
Het Piperidinyl oder Piperazinyl;
$R^1$ Wasserstoff;
$R^2$ Wasserstoff, Methyl, Phenyl oder 4-Chlorphenyl;
$R^3$ Benzyl, 4-Chlorbenzyl oder 1,3-Dihydrobenzimidazol-2-on-1-yl, bedeuten.

Erfindungsgemäß von herausragender Bedeutung sind die Verbindungen der allgemeinen Formel (I), in denen die Gruppe -Het-$R^3$ für

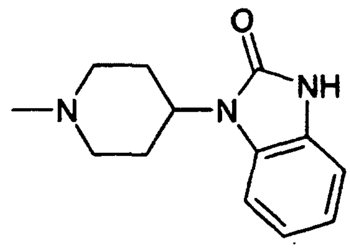

steht.

Als im Rahmen der vorliegenden Erfindung besonders bedeutsam sind die nachstehend genannten Verbindungen zu nennen:

- 1-{1-[2-(2-Methyl-imidazo[1,2-*a*]pyridin-3-yl)-ethyl]-piperidin-4-yl}-1,3-dihydrobenzimidazol-2-on;
- 1-[1-(3-Imidazo[1,2-*a*]pyridin-3-yl-propyl)-piperidin-4-yl]-1,3-dihydrobenzimidazol-2-on;
- 1-[1-(2-Imidazo[1,2-*a*]pyridin-3-yl-ethyl)-piperidin-4-yl]-1,3-dihydro-benzimidazol-2-on; 1-{1-[3-(2-Phenyl-imidazo [1,2-*a*]pyridin-3-yl)-propyl]-piperidin-4-yl}-1,3-dihydro-benzimidazol-2-on

Gegenstand der Erfindung sind die jeweiligen Verbindungen gegebenenfalls in Form der einzelnen optischen Isomeren, Mischungen der einzelnen Enantiomeren oder Racemate, in From der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

Als Alkylgruppen (auch soweit sie Bestandteil anderer Reste sind) werden soweit nicht anders definiert verzweigte und unverzweigte Alkylgruppen mit 1 bis 4 Kohlenstoffatomen betrachtet. Beispielsweise werden genannt: Methyl, Ethyl, Propyl und Butyl. Sofern nicht anders genannt, sind von den vorstehend genannten Bezeichnungen Propyl und Butyl sämtliche der möglichen isomeren Formen umfaßt. Dementsprechend umfaßt die Bezeichnung Propyl die beiden isomeren Reste n-Propyl und iso-Propyl und die Bezeichnung Butyl n-Butyl, iso-Butyl, sec. Butyl und tert. -Butyl. Gegebenfalls werden zur Bezeichnung der vorstehend genannten Alkylreste auch gängige Abkürzungen wie Me für Methyl, Et für Ethyl etc. verwendet.

Als Alkylengruppen werden verzweigte und unverzweigte Alkylenbrücken mit 1 bis 4 Kohelnstoffatomen betrachtet. Beispielsweise werden genannt: Methylen, Ethylen, Propylen und Butylen Sofern nicht anders genannt, sind von den vorstehend genannten Bezeichnungen Propylen und Butylen sämtliche der möglichen isomeren Formen umfaßt. Dementsprechend umfaßt die Bezeichnung Propylen die beiden isomeren Brücken n-Propylen und Dimethylmethylen und die Bezeichnung Butylen die isomeren Brücken n-Butylen, 1-Methylpropylen, 2-Methylpropylen, 1.1-Dimethylethylen und 1.2-Dimethylethylen.

Als Alkenylgruppen (auch soweit sie Bestandteil anderer Reste sind) werden verzweigte und unverzweigte Alkenylgruppen mit 2 bis 4 Kohlenstoffatomen genannt, soweit sie mindestens eine Doppelbindung aufweisen, wie zum Beispiel Vinyl (soweit keine unbeständigen Enamine oder Enolether gebildet werden), Propenyl, isoPropenyl und Butenyl.

Als Halogen wird im allgemeinen Fluor, Chlor, Brom oder Jod bezeichnet. Sofern nichts anderes angegeben ist, gilt Chlor als im Rahmen der vorliegenden Erfindung als bevorzugt.
"=O" bedeutet ein über eine Doppelbindung verknüpftes Sauerstoffatom.

In den vorstehehend genannten Definitionen sind entsprechend der allgemeinen Formel (I) alle der für die Reste A und Het angegebenen Definitionen als zweibindige Gruppen anzusehen. Die Reste A können, sofern es sich um die unsymmetrischen Brückenglieder $-CH_2-CO-$, $-CH_2-CH_2-CO-$ oder $-CH=CH-CO-$ handelt, in zwei verschiedenen Ausrichtungen mit ihren benachbarten Gruppen verknüpft sein. Als bevorzugte Ausrichtung wird erfindungsgemäß die Verknüpfung angesehen, bei der die Carbonylfunktion der vorstehend genannten Brückenglieder direkt an die Gruppe "Het" angebunden ist. Die Gruppe Het kann ebenfalls in verschiedenen Ausrichtungen mit ihren benachbarten Gruppen verknüpft sein. Als bevorzugte Ausrichtung wird erfindungsgemäß die Verknüpfung angesehen, bei der die Gruppe Het mit mindestens einem Stickstoffatom mit der Brücke A verknüpft sind.
Besonders bevorzugt sind die als Gruppen Het definierten Reste Piperazinyl und Dihydrobenzimidazolonyl ferner über ihr zweites N-Atom mit dem Rest $R^3$ verknüpft.

Unter Dihydrobenzimidazolonyl wird 1,3-Dihydro-benzimidazol-2-on-1-yl verstanden.

Ein weiterer Aspekt der vorliegenden Erfindung zielt auf die Verwendung der vorstehend definierten Verbindungen der allgemeinen Formel (I) als Arzneimittel. Insbesondere zielt die vorliegende Erfindung auf die Verwendung der Verbindungen der allgemeinen Formel (I) zur Herstellung eines Arzneimittels zur Vorbeugung und/oder Behandlung von Erkrankungen, in denen durch Eingreifen (bevorzugt Inhibition) in den Prozeß der Aβ-Entstehung oder Freisetzung aus Zellen, ein therapeutischer Nutzen erzielt werden kann. Erfindungsgemäß bevorzugt ist die vorstehend genannte Verwendung von Verbindungen der allgemeinen Formel (I) zur Herstellung eines Arzneimittels zur Vorbeugung und/oder Behandlung der Alzheimerschen Krankheit.

Ein synthetischer Zugang zu den erfindungsgemäßen Verbindungen der allgemeinen Formel (I) kann unter Anwendung unterschiedlicher Methoden, gegebenenfalls in Anlehnung an oder unter Verwendung von konventionellen chemischen Synthesemethoden wie nochfolgend detaillierter beschrieben erfolgen.

Eine mögliche synthetische Vorgehensweise zur Darstellung der Verbindungen der allgemeinen Formel (I) in denen A für eine Ethylenbrücke steht, ist aus Schema 1 ersichtlich.

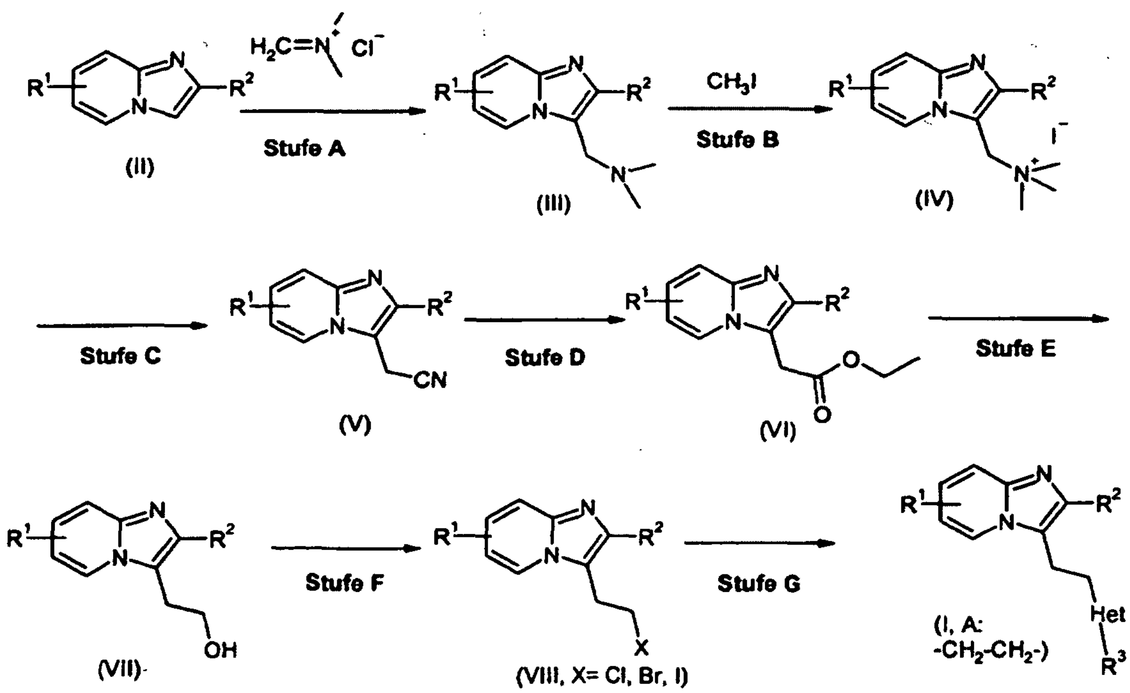


Schema 1:

In einem ersten Schritt (Stufe A) wird ein 2H-Imidazo[1,2-*a*]pyridin (II) durch Umsetzung mit N,N-Dimethylmethyleniminiumchlorid in die Aminomethylsubstituierten Verbindungen (III) überführt, aus denen sich mittels Alkylierung mit Methyliodid (Stufe B) die quartären Amoniumsalze (IV) erhalten lassen. Die lassen sich in die Nitrile (V) überführen (Stufe C), deren Hydrolyse Zugang zu den Carbonsäureestem (VI) gibt (Stufe D). Reduktion der Verbindungen (VI) nach Stufe E führt zu den Alkoholen (VII) die gemäß Stufe F in die Halogenide (VIII) überführt werden. Aus diesen können über nukleophile Substitution (Stufe G) die Verbindungen der Formel (I), in denen A $-CH_2-CH_2-$ bedeutet, erhalten werden.

Alternativ zu der in Schema 1 dargestellten Synthesestrategie lassen sich die Verbindungen der Formel (VII) auch gemäß Schema 2 erhalten.

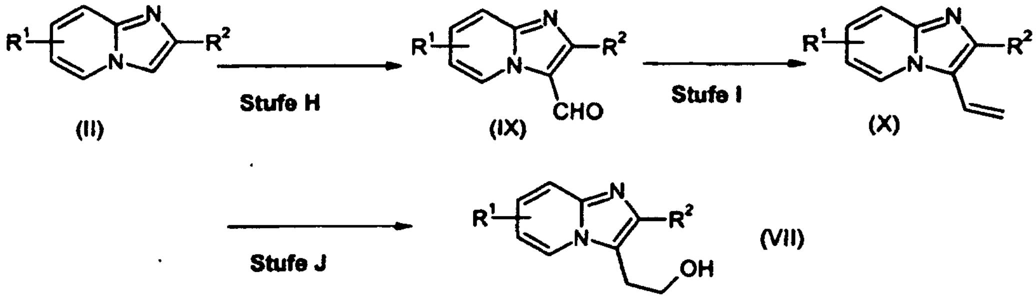


Schema 2:

Hierzu werden die 2H-Imidazo[1,2-*a*]pyridine (II) in einer Vilsmeier-Reaktion in die formylierten 2H-Imidazo [1,2-a]pyridine (IX) überführt (Stufe H). Aus diesen lassen sich mittels Wittig-Reaktion die Olefine (X) erhalten (Stufe I). Die Alkohole (VII) sind gemäß Stufe J durch Hydroborierung der Verbindungen (X) zugänglich.

Eine weitere Verfahrensvariente erlaubt einen von Schema 1 abweichenden Zugang zu den Verbindungen der Formel (VI). Dieser ist in Schema 3 skizziert.

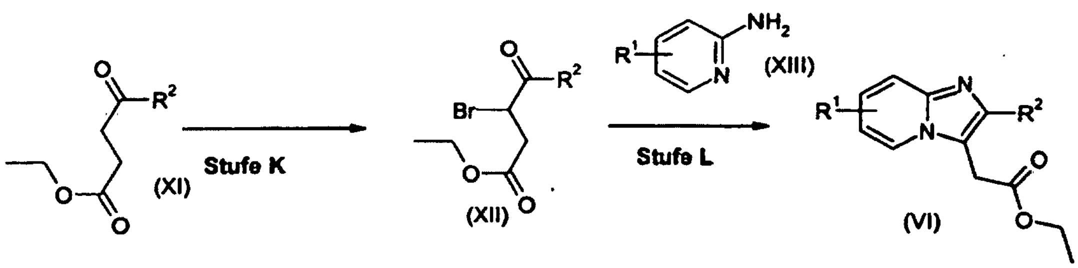


Schema 3:

Ausgehend von den Carbonylverbindungen (XI) lassen sich durch Bromierung die α-Bromcarbonylverbindungen (XII) erhalten (Stufe K) die nach Umsetzung mit 1-Aminopyridinen der Formel (XIII) einen Zugang zu den Estern (VI) eröffnen (Stufe L).

Aus den Estern der Formel (VI) können alternativ zu der in Schema (I) dargestellten Vorgensweise Verbindungen der Formel (I) erhalten werden, in denen A für die Brücke $-CH_2-CO-$ steht. Diese lassen sich ihrerseits wiederum in Verbindungen der Formel (I) überführen, in denen A $-CH_2-CH_2-$ bedeutet (Schema 4).

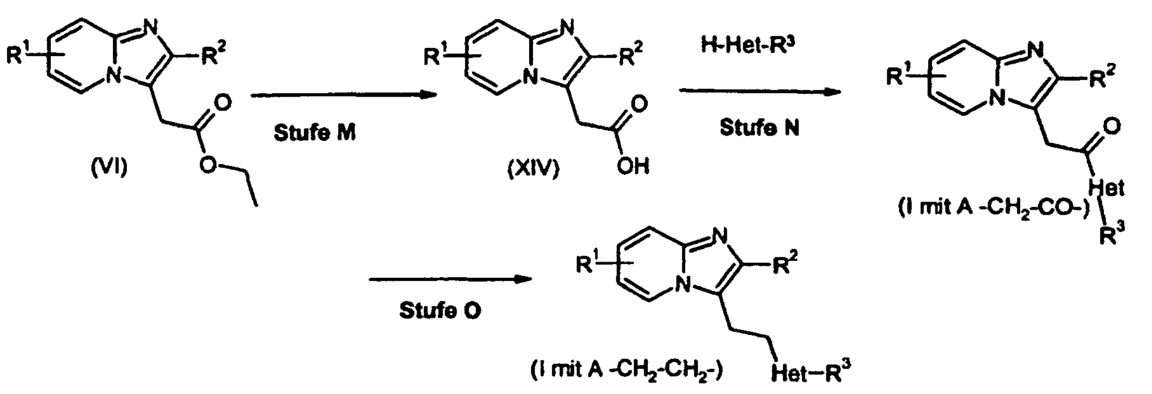


Schema 4:

Die Verseifung der Ester (VI) führt gemäß Stufe M zu den freien Carbonsäuren (XIV). Diese lassen sich

gegebenenfalls unter Verwendung von geeigneten Kupplungsreagenzien nach Stufe N durch Umsetzung mit den Aminen H-Het-R3 in die entsprechenden Amide überführen (I, A: $-CH_2-CO$), welche reduktiv in die entsprechenden Verbindungen der Formel (I) mit A gleich $-CH_2-CH_2-$ transformiert werden können (Stufe O).

Zur Herstellung von Verbindungen der Formel (I), in denen A $-CH_2-CH_2-CH_2-$, oder -CH=CH-CO- bedeutet, bietet sich die gemäß Schema 5 dargestellte Vorgensweise an.

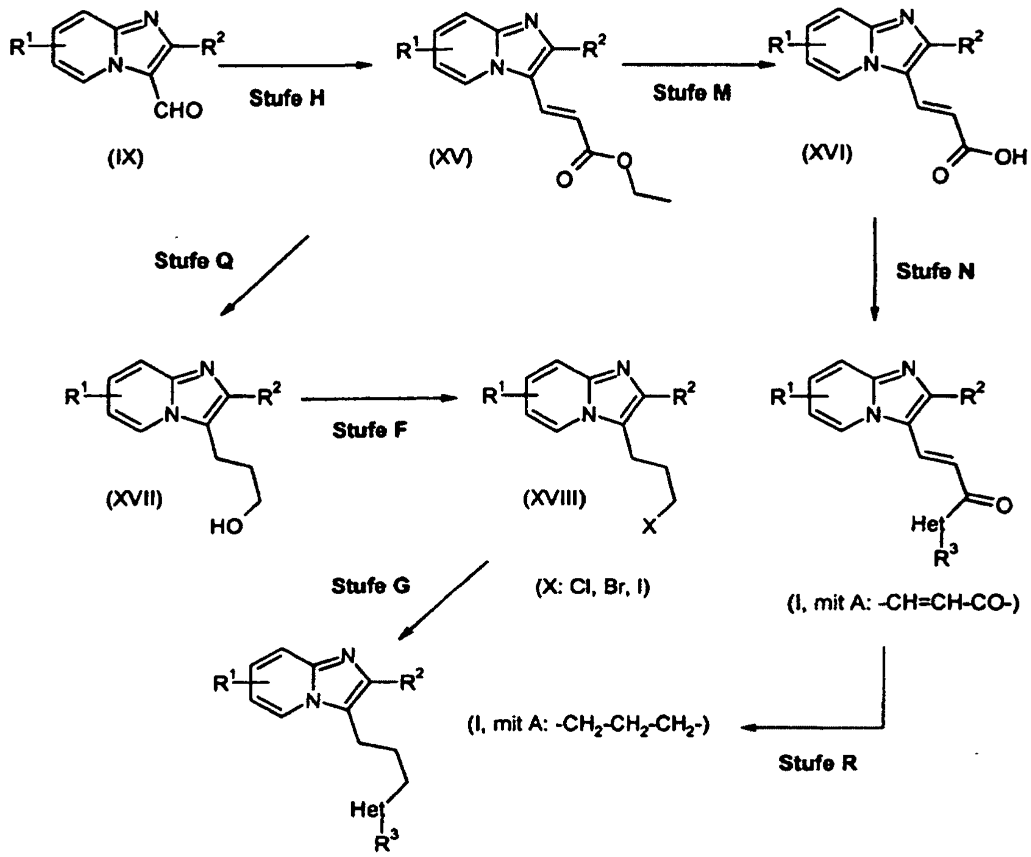


Schema 5:

Ausgehend von den Carbonylierten Verbindungen (IX) gelingt die Synthese der $\alpha,\beta$-ungesättigten Ester (XV) über eine Wittig- oder Wittig-Horner-Emmons-Reakation (Stufe H). Werden die Ester (XV) gemäß Stufe M zu den Carbonsäuren (XVI) verseift, so lassen sich diese sich nach Stufe N in die $\alpha,\beta$-ungesättigten Amide (I, mit A gleich -CH=CH-CO-) überführen. Aus diesen können reduktiv die Verbindungen der Formel (I) in denen A für Propylen steht, erhalten werden (Stufe R). Letztere sind alternativ zugänglich durch Reduktion (Stufe Q) der Ester (XV) zu den Alkoholen (XVII), welche nach Standardverfahren (Stufen F und G) über die Halogenide (XVIII) Zugang zu den Zielverbindungen liefern.

Gegebenenfalls kann bei den vorstehend beschriebenen Syntheseverfahren die Verwendung gängiger Schutzgruppen geboten erscheinen, die nach Standardverfahren sowohl eingeführt, als auch abgespalten werden können.

Die nachfolgenden Synthesebeispiele dienen lediglich der exemplarischen Erläuterung ohne den Gegenstand der Erfindung auf selbige zu beschränken.

Beispiel 1: 1-[1-(2-Imidazo[1,2-*a*]pyridin-3-yl-ethyl)-piperidin-4-yl]-1,3-dihydrobenzimidazol-2-on

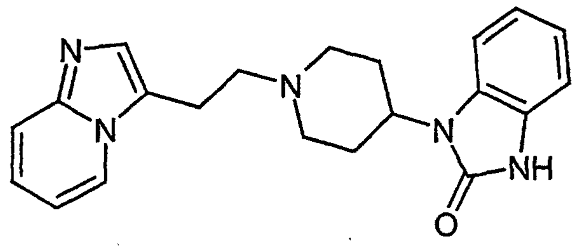

1.1.: 3- N,N-Dimethylaminomethyl-imidazo[1,2-*a*]pyridin (entspricht Verbindung der Formel (III)

***Verfahren nach Stufe A:***

13,0 g (110 mmol) 2*H*-Imidazo[1,2-*a*]pyridin in 120 ml Acetonitril werden mit 21,0 g (224 mmol) N,N-Dimethylmethyleniminiumchlorid versetzt und 6 h bei 65°C gerührt. Nach 16 h Stehen bei Raumtemperatur wird auf 70 ml konzentriert. Man saugt die ausgefallenen Kristalle ab, löst in 60 ml Wasser und stellt mit konz. NatriumhydroxidLösung alkalisch. Es wird mit Essigester extrahiert, die organischen Phasen werden getrocknet und eingeengt. Man erhält 19,2 g des Produktes als weiße Kristalle.

1.2.: 3- N,N,N-Trimethylammonium-methyl-imidazo[1,2-*a*]pyridin-iodid (entspricht Verbindung der Formel (IV)

***Verfahren nach Stufe B:***

19,2 g (110 mmol) der Verbindung Beispiel 1.1. werden in 120 ml Ethanol gelöst und mit 21,0 g (147 mmol) Methyliodid versetzt. Man rührt 4 h bei Raumtemperatur. Anschließend wird mit 50 ml Ether versetzt und mit einem Eisbad gekühlt. Nach Absaugen der Kristalle und Trocknen erhält man 31,0 g Produkt.

1.3.: 3-Cyanomethyl-imidazo[1,2-*a*]pyridin (entspricht Verbindung der Formel (V)

***Verfahren nach Stufe C:***

31,0 g (97,8 mmol) der Verbindung Beispiel 1.2. werden in 220 ml Ethanol mit 16,0 g (326 mmol) Natriumcyanid versetzt und 16 h unter Rückfuss gekocht. Man engt ein, versetzt mit 100 ml Wasser und extrahiert mit Essigester. Die organischen Phasen werden eingeengt und der Rückstand aus Toluol umkristallisiert. Nach Waschen mit Ether wird 6,40 g Produkt erhalten.

1.4.: 3-Ethoxycarbonylmethyl-imidazo[1,2-*a*]pyridin (entspricht Verbindung der Formel (VI)

***Verfahren nach Stufe D:***

15 ml konz. Schwefelsäure werden in 38 ml Ethanol eingetragen. 6,40 g (40,8 mmol) der Verbindung Beispiel 1.3 werden hinzu gegeben und das Gemisch 6 h unter Rückfluss erhitzt. Anschließend lässt man 16 h stehen. Man gibt auf 30 ml Eis/Wasser und stellt mit Kaliumcarbonat vorsichtig auf pH 10. Es wird mit Essigester extrahiert, die vereinigten organischen Phasen getrocknet und eingeengt. Der Rückstand wird dreimal mit Ether ausgekocht und 5,50 g des Produktes durch vorsichtiges Einengen ausgefällt.

1.5.: 3-(2-Hydroxyethyl)-imidazo[1,2-*a*]pyridin (entspricht Verbindung der Formel (VII))

***Verfahren nach Stufe* E:**

Eine Lösung von 3,30 g (29,7 mmol) wasserfreiem Calciumchlorid in 35 ml Ethanol wird mit einer Lösung von 5,50 g (27,0 mmol) der Verbindung Beispiel 1.4, gelöst in 50 ml THF, versetzt. Man kühlt auf -10°C und gibt portionsweise 2,26 g (59,7 mmol) Natriumborhydrid zu. Man lässt auf Raumtemperatur erwärmen, rührt 6 h und lässt dann 16

h bei 4°C stehen. Das Gemisch wird mit 2 N HCl versetzt und anschließend mit konz. Ammoniaklösung wieder alkalisch gestellt. Das organische Lösungsmittel wird abdestilliert und die wässrige Phase wird mit Kaliumcarbonat auf pH 11 gestellt. Extraktion mit Essigester, Einengen und Trocknen bei 40°C ergibt 3,80 g gelbe Kristalle.

Verfahrensvarianten zur Herstellung der Verbindungen der allgemeinen Formel (VII) gemäß Stufen H, I und J:

<u>1.5.1.: 3-Formyl-imidazo[1,2-*a*]pyridin (entspricht Verbindung der Formel (IX))</u>

***Verfahren nach Stufe H:***

Zu 70,4 g (846 mmol) DMF werden unter Eiskühlung innerhalb von 50 min 64,9 g (423 mmol) Phosphoroxichlorid so zugetropft, dass die Temperatur bei 2-5°C bleibt. Das Gemisch wird 1 h bei 5°C gerührt und anschließend tropfenweise mit 25,0 g (212 mmol) 2*H*-Imidazo[1,2-*a*]pyridin in 30 ml DMF bei 5 -10°C versetzt. Anschließend wird 1 h bei 5°C gerührt und dann 6 h auf 100°C erwärmt. Danach wird unter Eiskühlung 100 ml Wasser zugetropft und mit 40 ml konz. Ammoniaklösung auf pH 7 gestellt. Man extrahiert erschöpfend mit Dichlormethan, wäscht die organische Phase mit Wasser, trocknet und engt ein. Nach Reinigung des Rohproduktes durch Flash-Chromatographie (Dichlormethan/Ethanol 95:5) werden 11,3 g Produkt erhalten.

<u>1.5.2.: 3-Vinyl-imidazo[1,2-*a*]pyridin (entspricht Verbindung der Formel (X))</u>

***Verfahren nach Stufe I:***

Man legt 1,00 g (6,89 mmol) der Verbindung Beispiel 1.5.1 gelöst in 30 ml THF vor und gibt 3,70 g (10,4 mmol) Methyltriphenylphosphoniumbromid zu. Nach 10 min werden 3,01 g Kaliumtertiärbutylat gelöst in 35 ml THF zugetropft, wobei sich das Reaktionsgemisch etwas erwärmt. Das Reaktionsgemisch wird 4 h bei Raumtemperatur gerührt, mit 10 ml Wasser versetzt und die klare Lösung 10 min gerührt. Die organische Phase wird abgetrennt und die wässrige mit Dichlormethan extrahiert. Man trocknet, engt ein und reinigt den Rückstand durch Flash-Chromatographie (Dichlormethan/Ethanol 95:5) und erhält so 480 mg kristallines Produkt.

<u>1.5.: 3-(2-Hydroxyethyl)-imidazo[1,2-*a*]pyridin (entspricht Verbindung der Formel (VII))</u>

***Verfahren nach Stufe J:***

Zu 4,37 ml (8,74 mmol) einer 2 M Lösung von Borandimethylsulfid-Komplex in THF werden unter Schutzgas 735 mg (8,74 mmol) 2,3-Dimethyl-2-buten zugetropft. Anschließend lässt man 2,5 h bei 5 - 10°C rühren und tropft diese Lösung zu 420 mg (2,91 mmol) der Verbindung Beispiel 1:5.2 gelöst in 15 ml THF und 15 ml Dichlormethan. Man lässt 16 h reagieren und versetzt unter Kühlung mit 4,7 ml Ethanol, dann mit 13 ml 2 Natriumhydoxidlösung und dann mit 6,3 ml Wasserstoffperoxidlösung (30%ig). Anschließend wird 3 h bei Raumtemperatur rühren gelassen. Die organische Phase wird abgetrennt und die wässrige mit Dichlormethan extrahiert. Die vereinigten organischen Phasen werden getrocknet und eingeengt. Man reinigt durch Flash-Chromatographie (Gradient: Dichlormethan/Ethanol 90:10 ->70:30) und erhält so 136 mg Produkt.

<u>1.6.: 3-(2-Bromethyl)-imidazol[1,2-*a*]pyridin (entspricht Verbindung der Formel (VIII))</u>

***Verfahren nach Stufe F:***

2,31 g (14,3 mmol) der Verbindung Beispiel 1.5 werden in 20 ml Chloroform gelöst. Unter Eiskühlung setzt man innerhalb von 10 min tropfenweise 4,00 g (19,2 mmol) Thionylbromid zu. Nachdem zunächst 4 h unter Eiskühlung gerührt wurde, lässt man weitere 16 h bei Raumtemperatur rühren. Man versetzt mit 20 ml THF und vervollständigt die Fällung nach 10 min durch Zugabe von 20 ml Ether. Nach 30 min Rühren im Eisbad wird abzentrifugiert. Umfällen des Kristallisats ergibt 2,35 g Rohprodukt als Hydrobromid.

<u>1.7.: 1-[1-(2-Imidazo[1,2-*a*]pyridin-3-yl-ethyl)-piperidin-4-yl]-1,3-dihydro-benzimidazol-2-on</u>

***Verfahren nach Stufe G:***

2,35 g (6,54 mmol) des Hydrobromids Beispiel 1.6 werden in 30 ml DMF und 70 ml Aceton gelöst und mit 1,43 g (6.58 mmol) 1-Piperidin-4-yl-1,3-dihydro-benzimidazol-2-on, 2,80 g (20,3 mmol) Kaliumcarbonat und 500 mg (3,01 mmol) Kaliumiodid versetzt und 7 h auf 55°C erwärmt. Anschließend lässt man noch 16 h bei Raumtemperatur

rühren. Das Lösungsmittel wird entfernt und der Rückstand über Flash-Chromatographie (Dichlormethan/Ethanol 90:10) gereinigt. Anschließend wird aus Dichlormethan/Ethanol 90:10 umkristallisiert. 750 mg freie Base werden in 10 ml Ethanol gelöst und mit 3 ml etherischer Salzsäure versetzt. Durch Umkristallisieren aus Methanol und trocknen bei 65°C i. Vac. erhält man 520 mg Produkt als Hydrochlorid. MS: m/z 362 [$(M+H)^+$]; Schmp.: 197°C;

**Beispiel 2: 1-[1-[2-(2-Methyl-imidazo[1,2-*a*]pyridin-3-yl)-ethyl]-piperidin-4-yl]-1,3-dihydro-benzimidazol-2-on**

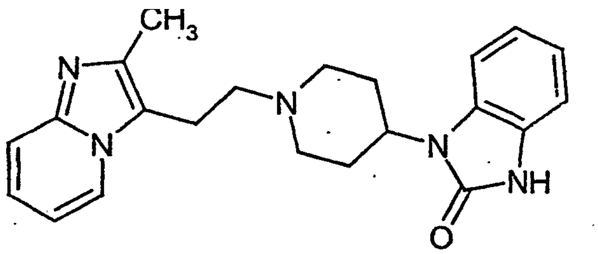

Herstellung analog Beispiel 1; Schmp.: 194°C

**Beispiel 3: 3-{2-[4-(2-Hydroxyethyl)-piperidin-1-yl]ethyl}-2-methyl-imidazo[1,2-*a*]pyridin**

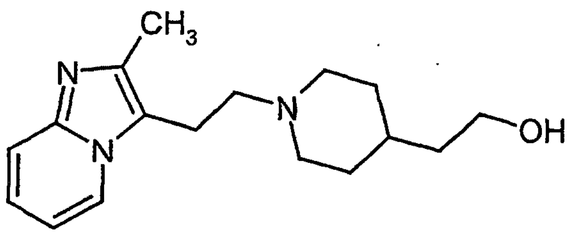

Herstellung analog Beispiel 1; Schmp.: Öl; MS: m/z 288 [$(M+H)^+$];

**Beispiel 4: 1-[2-(2-Methyl-imidazo[1,2-*a*]pyridin-3-yl)-ethyl]-3-(propen-2-yl)-1,3-dihydro-benzimidazol-2-on**

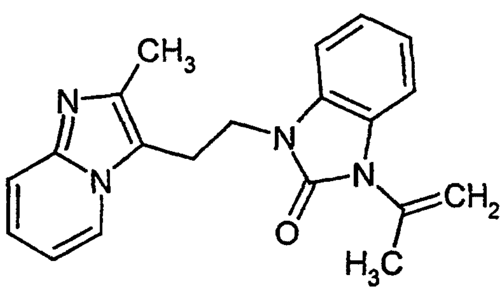

Herstellung analog Beispiel 1; Schmp.: 112°C;

**Beispiel 5: 1-[2-(2-Methyl-imidazo[1,2-*a*]pyridin-3-yl)-ethyl]-1,3-dihydrobenzimidazol-2-on**

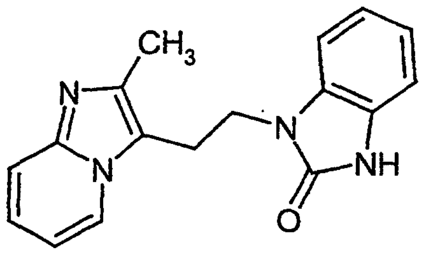

70 mg (0,21 mmol) von Beispiel 4 werden in 2 ml Ethanol gelöst und mit 100 µl ethanolischer Salzsäure versetzt. Man rührt 3 h bei Raumtemperatur, entfernt das Lösungsmittel und gewinnt 62,4 mg des Produktes durch Kristallisation aus Dichlormethan/Ethanol. Schmp.: 236°C;

**Beispiel 6: 3-{2-[4-(2-N-Morpholino-ethyl)-piperazin-1-yl]-2-oxo-ethyl}-2-phenylimidazo[1,2-*a*]pyridin**

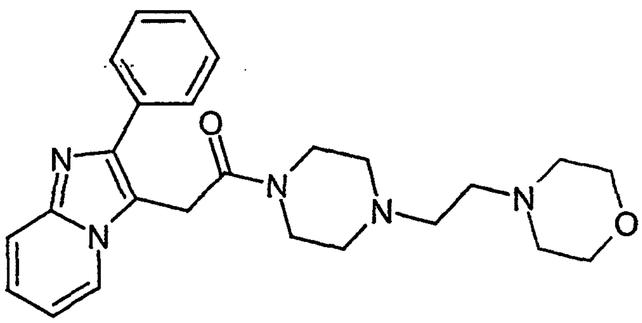

6.1.: 3- Carboxymethyl-2-phenyl-imidazo[1,2-*a*]pyridin (entspricht Verbindung der Formel (XIV)

***Verfahren nach Stufe M:***

3,50 g (12,5 mmol) 3-Ethoxycarbonylmethyl-2-phenyl-imidazo[1,2-*a*]pyridin (Verbindung nach Formel (VI) erhältlich nach Stufe D) werden in 200 ml 2 N Salzsäure 5 h auf 100°C erwärmt. Man destilliert die Salzsäure ab, nimmt den Rückstand in Wasser auf und bringt durch Zugabe von konz. Ammoniaklösung zur Kristallisation. So erhält man 2,60 g Produkt. Schmp.: Zers. 243 - 244°C;

6.2.: 3-{2-[4-(2-N-Morpholino-ethyl)-piperazin-1-yl]-2-oxo-ethyl}-2-phenyl-imidazo[1,2-*a*]pyridin

***Verfahren nach Stufe N:***

230 mg (0,99 mmol) der Verbindung Beispiel 6.1 werden in 15 ml THF und 15 ml Dichlormethan gelöst und mit 217 mg (0,99 mmol) 4-(2-Piperazin-1-yl-ethyl)-morpholin versetzt. Man gibt 350 mg (0,99 mmol) TBTU und 128 mg (0,99 mmol) DIPEA hinzu und rührt 24 h bei Raumtemperatur. Das Lösungsmittel wird entfernt, der Rückstand in Essigester aufgenommen und mit 10proziger Natriumhydrogencarbonatlösung, gesättigter Natriumchloridlösung und Wasser gewaschen. Die organische Phase wird getrocknet, eingeengt und das Rohprodukt mit Diethylether/Diisopropylether verrieben. So werden 341 mg des Produktes erhalten. Schmp.: 136 - 138°C;

**Beispiel 7: 1-{1-[2-(2-Phenyl-imidazo[1,2-*a*]pyridin-3-yl)-2-oxo-ethyl]-piperidin-4-yl}-1,3-dihydro-benzimidazol-2-on**

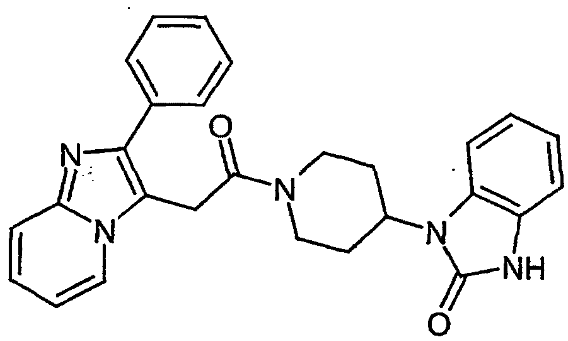

Herstellung analog Beispiel 6; MS: m/z 452 [(M+H)$^+$]; Schmp.: 263 - 265°C;

**Beispiel 8: 1-[2-(2-Phenyl-imidazo[1,2-*a*]pyridin-3-yl)-2-oxo-ethyl]-3-(propen-2-yl)-1,3-dihydro-benzimidazol-2-on**

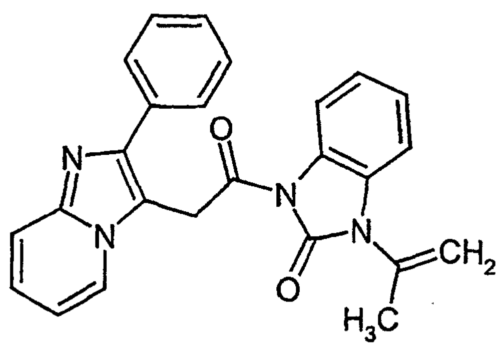

Herstellung analog Beispiel 6; MS: m/z 409 [(M+H)$^+$]; Schmp.: 167°C;

**Beispiel 9: 3-{2-[4-(2-N-Morpholino-ethyl)-piperazin-1-yl]-ethyl}-2-phenylimidazo[1,2-*a*]pyridin**

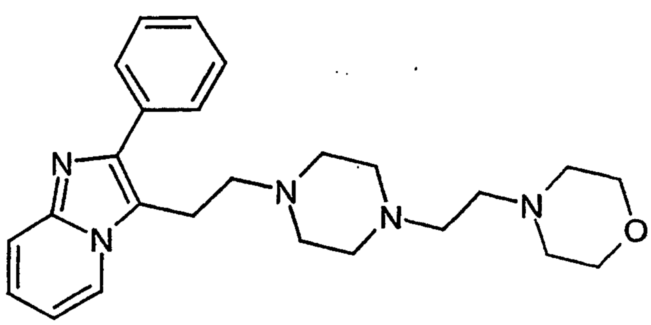

***Verfahren nach Stufe O:***

130 mg (0.30 mmol) der Verbindung Beispiel 6, gelöst in 10 ml THF, werden mit 14 mg (0,30 mmol) Lithiumaluminiumhydrid versetzt und 12 h bei Raumtemperatur gerührt. Die Lösung wird vom Niederschlag abgetrennt, der Niederschlag mit Essigester ausgewaschen und die vereinigten organischen Lösungen eingeengt und flash-chromatographisch (Dichlormethan/Ethanol 90:10) gereinigt, um 56 mg Produkt zu erhalten. MS: m/z 420 [$(M+H)^+$];

**Beispiel 10: 1-[2-(2-Phenyl-imidazo[1,2-*a*]pyridin-3-yl)-2-oxo-ethyl]-1,3-dihydrobenzimidazol-2-on**

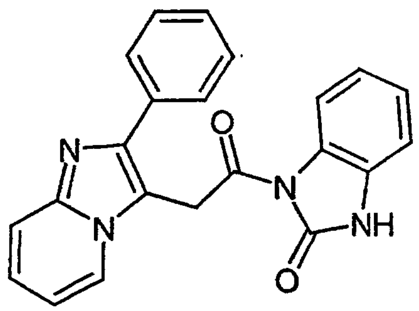

185 mg (0,50 mmol) der Verbindung Beispiel 8 werden in 3 ml Ethanol vorgelegt und mit 0,36 ml ethanolischer Salzsäure versetzt. Man lässt 72 h rühren, entfernt das Lösungsmittel und verreibt den kristallinen Feststoff mit Dichlormethan/Ethanol 95:5 um 120 mg des Produktes zu erhalten. MS: m/z 396 [$(M+H)^+$]; Schmp. 233 - 235°C;

**Beispiel 11: 1-[1-(3-Imidazo[1,2-*a*]pyridin-3-yl-propyl)-piperidin-4-yl]-1,3-dihydro-benzimidazol-2-on**

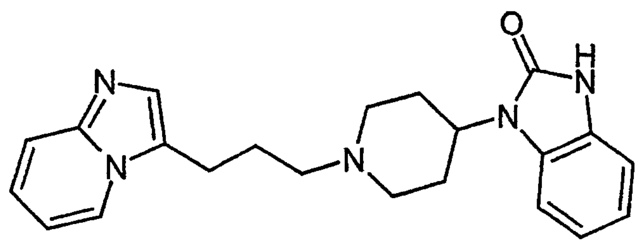

11.1.: 3-(2-Ethoxycarbonyl-(*E*)-vinyl)-imidazo[1,2-*a*]pyridin (entspricht Verbindung der Formel (XV))

***Verfahren nach Stufe H:***

7,60 g Natriumtertiäramylat werden in 420 ml Toluol vorgelegt, auf -20°C gekühlt und 15,4 g (68,8 mmol) in 200 ml Toluol gelöster Phosphonoessigsäuretriethylester innerhalb von 30 min zugetropft. Man rührt 2,5 h bei Raumtemperatur, kühlt auf -5°C ab und tropft 5,00 g (34,2 mmol) der in 210 ml Toluol gelösten Verbindung Beispiel 1.5.1 innerhalb von 30 min zu. Danach wird 4 h unter Rückfluss erhitzt. Anschließend wird das Lösungsmittel entfernt, der Rückstand in Essigester aufgenommen, mit Wasser gewaschen, die organische Phase getrocknet und eingeengt. Das Rohprodukt wird mit Isopropylether ausgerührt und ergibt 5,10 g kristallines Produkt. Schmp. 128°C;

11.2.: 3-(3-Hydroxypropyl)-imidazo[1,2-*a*]pyridin (entspricht Verbindung der Formel (XVII))

***Verfahren nach Stufe Q:***

290 mg (2,61 mmol) Calciumchlorid werden in 10 ml Ethanol gelöst und mit einer Lösung von 500 mg (2,31 mmol) der Verbindung Beispiel 11.1 in 15 ml THF versetzt. Man kühlt auf -15°C und gibt 250 mg (6,60 mmol) Natriumborhydrid portionsweise zu. Es wird zunächst 3 h bei 0°C gerührt und dann 72 h bei 4°C stehen gelassen. Die

Suspension wird mit 2 N Salzsäure versetzt und anschließend mit konz. Ammoniaklösung alkalisch gestellt. Die organischen Lösungsmittel werden abdestilliert und der Rückstand mit ges. Kaliumcarbonatlösung versetzt. Man extrahiert mit Essigester, trocknet und engt ein. Das Rohprodukt wird flash-chromatographisch (Dichlormethan/Ethanol 95:5) gereinigt, wodurch man 195 mg der gewünschten Verbindung als gelbliches Öl erhält. MS: m/z 177 [$(M+H)^+$];

11.3.: 3-(3-Brompropyl)-imidazo[1,2-*a*]pyridin (entspricht Verbindung der Formel (XVIII))

***Verfahren nach Stufe F:***

195 mg (1,10 mmol) der Verbindung Beispiel 11.2 werden in 2 ml Chloroform gelöst und unter Eiskühlung mit 315 mg (1,50 mmol) Thionylbromid in 5 Portionen versetzt. Man rührt 4 h bei 0°C, versetzt mit Dichlormethan und engt ein. Der Rückstand wird zwischen Wasser und Dichlormethan verteilt, mit 1 N NaOH auf pH 7 gestellt und mit Dichlormethan extrahiert. Die vereinigten organischen Phasen werden getrocknet und eingeengt. Man erhält 240 mg eines langsam kristallisierenden Öls.

11.4.: 1-[1-(3-Imidazo[1,2-*a*]pyridin-3-yl-propyl)-piperidin-4-yl]-1,3-dihydrobenzimidazol-2-on

***Verfahren nach Stufe G:***

240 mg (2,21 mmol) 1-Piperidin-4-yl-1,3-dihydro-benzimidazol-2-on werden in 3,5 ml Acetonitril und 3,5 ml DMF bei 50°C gelöst und bei dieser Temperatur eine Lösung von 240 mg (1,00 mmol) der Verbindung Beispiel 11.3 in 7,5 ml Essigester zugetropft. Das Gemisch wird zunächst 5 h bei 50°C, dann 72 h bei RT gerührt. Die Lösung wird eingeengt und der Rückstand flash-chromatographisch (Dichlormethan/Ethanol 90:10) gereinigt, wodurch man 48 mg Produkt erhält.
MS: m/z 376 [$(M+H)^+$]; Schmp.: 185°C;

**Beispiel 12: 1-{1-[3-(2-Phenyl-imidazo[1,2-*a*]pyridin-3-yl)-propyl]-piperidin-4-yl}-1,3-dihydro-benzimidazol-2-on**

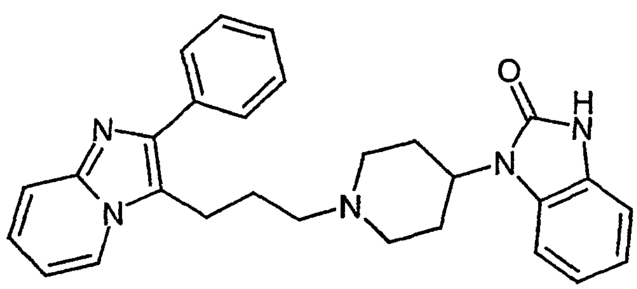

Herstellung analog Beispiel 11; Schmp.: 98°C;

**Beispiel 13: 1-{1-[3-(2-p-Chlorphenyl-imidazo[1,2-*a*]pyridin-3-yl)-propyl]piperidin-4-yl}-1,3-dihydro-benzimidazol-2-on**

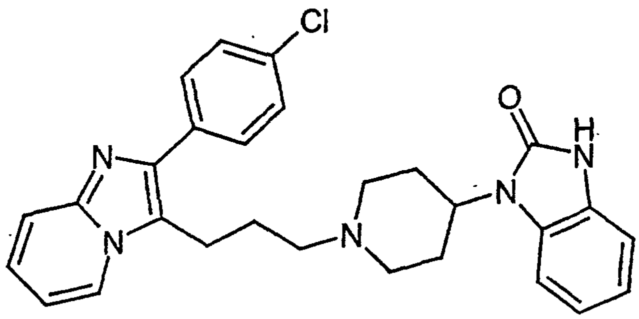

13.1.: 5-(4-Chlorphenyl)-5-oxo-4-brom-pentansäure-ethylester (entspricht Verbindung nach Formel (XX))

***Verfahren nach Stufe K:***

5,00 g (19,6 mmol) 5-(4-Chlorphenyl)-5-oxo-pentansäureethylester (entspricht Verbindung nach Formel (XIX)) werden in 50 ml Ether gelöst vorgelegt und bei -15°C 4,00 g (25,0 mmol) Brom zugetropft. Man kocht 2 h unter Rückfluss. Das Reaktionsgemisch wird mit Wasser gewaschen, die organische Phase getrocknet und eingeengt. Man erhält 5,90 g des Produktes als hellbraunes Öl.

13.2.: 3-(2-Ethoxycarbonyl-ethyl)-2-(4-chlomhenyl)-imidazo[1,2-*a*]pyridin (entspricht Verbindung der Formel (XXI))

***Verfahren nach Stufe L:***

3,29 g (35,0 mmol) 2-Aminopyridin (entspricht Verbindung nach Formel (XIII) werden vorgelegt und mit 5,90 g (17,7 mmol) der Verbindung Beispiel 13.1 versetzt. Man kocht 2,5 h unter Rückfluss und entfernt anschließend das Lösungsmittel. Der Rückstand wird zwischen Ether und Wasser verteilt, die organische Phase mit Wasser gewaschen und mit 2 N Salzsäure extrahiert. Man stellt die wässrige Phase mit konz. Ammoniaklösung alkalisch und extrahiert mit Ether. Die vereinigten organischen Phasen werden getrocknet, eingeengt und der Rückstand mit Diisopropylether verrieben. So erhält man 1,60 g des Produktes als kristallinen Feststoff.

13.3.: 3-(3-Hydroxypropyl)-2-(4-chlorphenyl)-imidazo[1,2-*a*]pyridin (entspricht Verbindung der Formel (XVII))

***Verfahren nach Stufe E:***

1,00 g (9,01 mmol) Calciumchlorid werden in 10 ml Ethanol gelöst und eine Lösung von 1,60 g (4,87 mmol) des Produktes aus Stufe B in 16 ml THF zugegeben. Man kühlt auf -10°C und gibt 450 mg (11,9 mmol) Natriumborhydrid portionsweise zu. Man lässt auf 0°C erwärmen rührt 6 h bei dieser Temperatur und lässt dann 72 h bei 4°C stehen. Man versetzt mit 2 N Salzsäurelösung und stellt mit konz. Ammoniaklösung alkalisch. Die organischen Lösungsmittel werden abdestilliert und die wässrige Phase mit Kaliumcarbonat auf pH 12 eingestellt. Extraktion mit Essigester, trocknen der vereinigten organischen Phasen und Einengen liefert 750 mg Produkt.

13.4.: 1-{1-[3-(2-p-Chlorphenyl-imidazo[1,2-*a*]pyridin-3-yl)-propyl]-piperidin-4-yl}-1,3-dihydro-benzimidazol-2-on

Ausgehend von Verbindung Beispiel 13.3. gelingt die Synthese der Titelverbindung in Analogie zur Vorgehensweise nach Beispiel 11.3 (Verfahren nach Stufe F) und anschließend nach Beispiel 11.4 (Verfahren nach Stufe G). Schmp.: 236°C;

**Beispiel 14: 3-[3-(4-Benzyl-piperazin-1-yl)-propyl]-2-(4-chlorphenyl)-imidazo[1,2-*a*]pyridin**

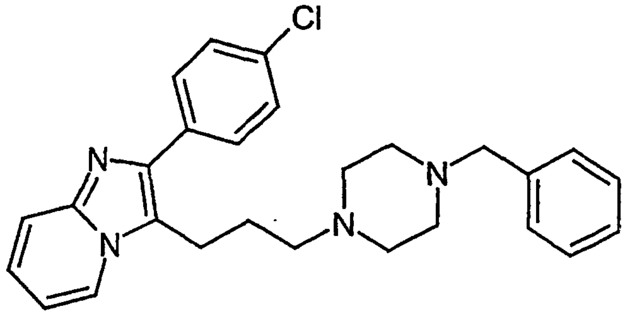

Herstellung analog Beispiel 13; Schmp. 174°C;

**Beispiel 15: 3-[3-(4-Phenyl-piperazin-1-yl)-propyl]-2-(4-chlorphenyl)-imidazo[1,2-*a*]pyridin**

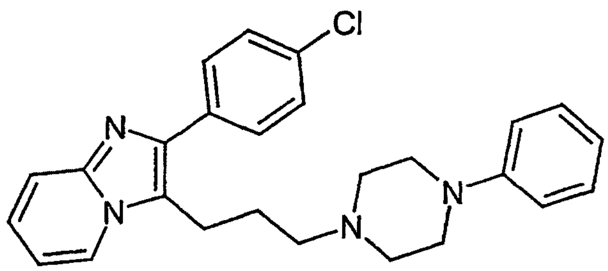

Herstellung analog Beispiel 13; Schmp. 169°C;

**Beispiel 16: 3-{3-[4-(4-Trifluorphenyl)-piperazin-1-yl]-propyl}-2-(4-chlorphenyl)-imidazo[1,2-*a*]pyridin**

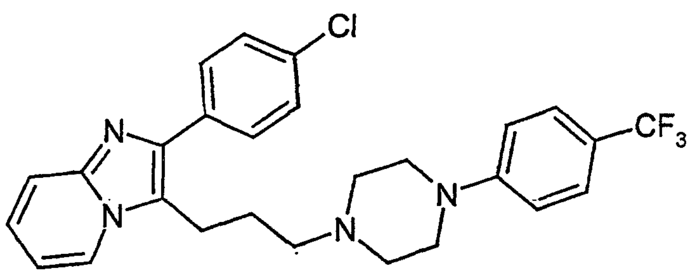

Herstellung analog Beispiel 13; Schmp. 174°C;

**Beispiel 17: 3-{3-[4-(4-Methylphenyl)-piperazin-1-yl]-propyl}-2-(4-chlorphenyl)-imidazo[1,2-*a*]pyridin**

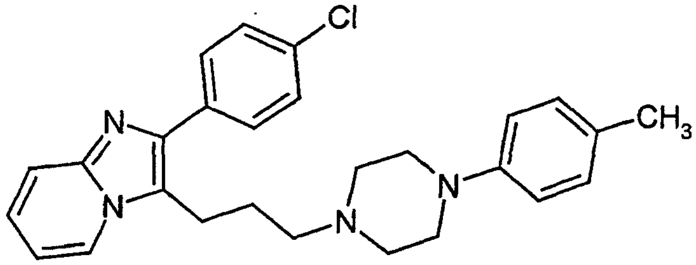

Herstellung analog Beispiel 13; Schmp. 154°C;

**Beispiel 18: 3-{3-[4-(2-Phenylethyl)-piperazin-1-yl]-propyl}-2-(4-chlorphenyl)imidazo[1,2-*a*]pyridin**

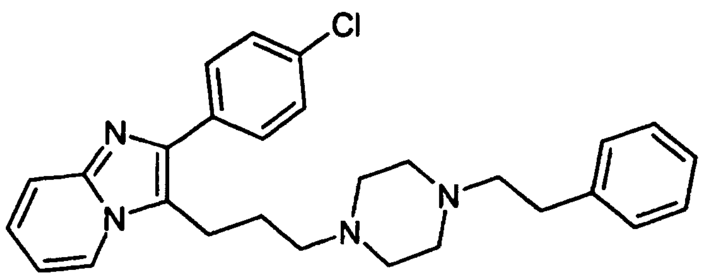

Herstellung analog Beispiel 13; Schmp. 156°C;

**Referenz-Beispiel 19: 3-{3-[4-(4-Chlorbenzyl)-piperazin-1-yl]-3-oxo-propyl}-2-(4-chlorphenyl)-imidazo[1,2-*a*] pyridin**

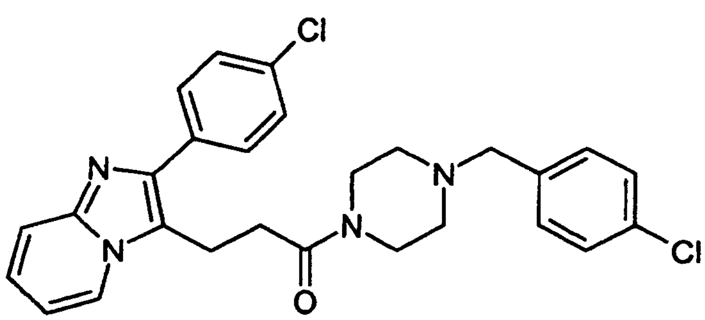

19.1.: 3-Carboxypropyl-2-(4-chlorphenyl)-imidazo[1,2-*a*]pyridin (entspricht Verbindung der Formel (XXII))

***Verfahren nach Stufe* M:**

3,50 g (10,7 mmol) der Verbindung Beispiel 13.2 in 35 ml Ethanol werden zusammen mit 12 ml 40proz. Natriumhydroxidlösung 2 h unter Rückfluss erhitzt und anschließend das Lösungsmittel entfernt. Der Rückstand wird in 400 ml Wasser gelöst. Man stellt mit konz. Salzsäure auf pH 1, trennt die ausgefallenen Kristalle ab und trocknet i.

Vac. um 3,03 g Produkt zu erhalten.

19.2.: 3-{3-[4-(4-Chlorbenzyl)-piperazin-1-yl]-3-oxo-propyl}-2-(4-chlorphenyl)-imidazo[1,2-*a*]pyridin

***Verfahren nach Stufe N:***

300 mg (1,00 mmol) der Verbindung Beispiel 19.1 werden in 7,5 ml THF und 7,5 ml Dichlormethan gelöst und mit 211 mg (1,00 mmol) 1-(4-Chlorbenzyl)-piperazin versetzt. Man gibt 353 mg (1,10 mmol) TBTU und 129 mg (1,00 mmol) DIPEA hinzu und rührt 16 h bei Raumtemperatur. Das Lösungsmittel wird entfernt, der Rückstand in Essigester aufgenommen und mit 10proz. Natriumhydrogencarbonatlösung, gesättigter Natriumchloridlösung und Wasser gewaschen. Die organische Phase wird getrocknet, eingeengt und das Rohprodukt mit Diisopropylether verrieben. So werden 190 mg des Produktes erhalten. Schmp.: 159°C;

**Beispiel 20: 3-{3-[4-(4-Chlorbenzyl)-piperazin-1-yl]-propyl}-2-(4-chlorphenyl)imidazo[1,2-*a*]pyridin**

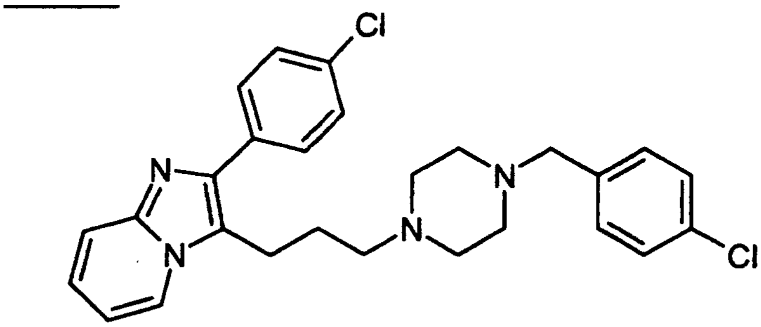

***Verfahren nach Stufe O:***

160 mg (0,32 mmol) der Verbindung Referenz-Beispiel 19 werden in 10 ml THF gelöst und unter Eiskühlung werden 4 ml (4 mmol) einer 1 M Lösung von Borantetrahydrofuran-Komplex in THF zugegeben. Man rührt 3 h unter Eiskühlung und versetzt mit 8 ml 1 N Salzsäure. Anschließend wird mit 10 proz. Natriumhydrogencarbonatlösung auf pH 8 gestellt. Man engt ein, und extrahiert den Rückstand mit Essigester. Trocknen und flash-chromatographische Reinigung (Dichlormethan/Ethanol 95:5) ergibt 10 mg des Produktes.

**Beispiel 21: 1-[3-(2-Phenyl-imidazo[1,2-*a*]pyridin-3-yl)-1-oxo-2(*E*)propenyl]-piperidin-4-yl}-1,3-dihydro-benzimidazol-2-on**

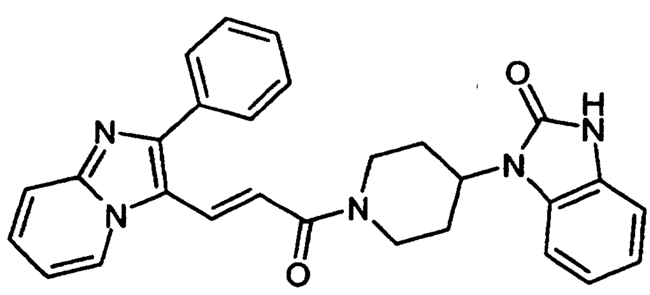

21.1.: 3-(2-Carboxy-(*E*)-vinyl)-2-phenyl-imidazo[1,2-*a*]pyridin (entspricht Verbindung der Formel (XVI))

***Verfahren nach Stufe M:***

0,90 g 3-(2-Ethoxycarbonyl-(*E*)-vinyl)-2-phenyl-imidazo[1,2-*a*]pyridin (erhältlich nach Verfahren Stufe H analog zu Beispiel 11.1) in 10 ml Ethanol werden mit 3 ml 40proz. Natriumhydroxidlösung versetzt und 2 h unter Rückfluss erhitzt. Man lässt abkühlen und trennt die ausgefallenen Kristalle ab. Diese werden in 10 ml Wasser gelöst und unter Eiskühlung wird tropfenweise konz. Salzsäure zugegeben. Die ausgefallenen Kristalle werden mit Wasser gewaschen und getrocknet, um 438 mg Produkt zu erhalten.

21.2.: 1-[3-(2-Phenyl-imidazo[1,2-*a*]pyridin-3-yl)-1-oxo-2(*E*)propenyl]-piperidin-4-yl}-1,3-dihydro-benzimidazol-2-on

***Verfahren nach Stufe N:***

200 mg (0,81 mmol) der Verbindung Beispiel 21.2 werden in 10 ml THF und 10 ml Dichlormethan gelöst und mit 175 mg (0,81 mmol) 1-Piperidin-4-yl-1,3-dihydrobenzimidazol-2-on versetzt. Man gibt 289 mg (0,90 mmol) TBTU und 116 mg (0,90 mmol) DIPEA hinzu und rührt 20 h bei Raumtemperatur. Das Lösungsmittel wird entfernt, der Rückstand in Essigester aufgenommen und mit 10proz. Natriumhydrogencarbonatlösung, gesättigter Natriumchloridlösung und Wasser gewaschen. Die organische Phase wird getrocknet, eingeengt und das Rohprodukt mit Diethylether verrieben. So werden 136 mg des Produktes erhalten.
Schmp.: > 230°C;

**Referenz-Beispiel 22: 3-{3-[4-(4-Chlorbenzyl)-piperazin-1-yl]-1-oxo-2(*E*)-propenyl}-2-phenyl-6-chlor-imidazo [1,2-*a*]pyridin**

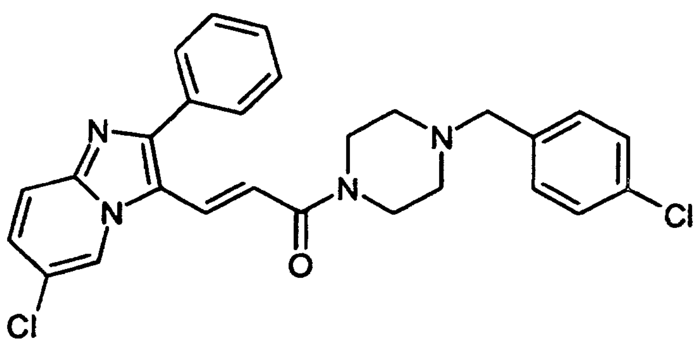

Herstellung analog Beispiel 21; Schmp.: 104°C;

**Beispiel 23: 1-[3-(Imidazo[1,2-*a*]pyridin-3-yl)-1-oxo-2(*E*)propenyl]-piperidin-4-yl}-1,3-dihydro-benzimidazol-2-on**

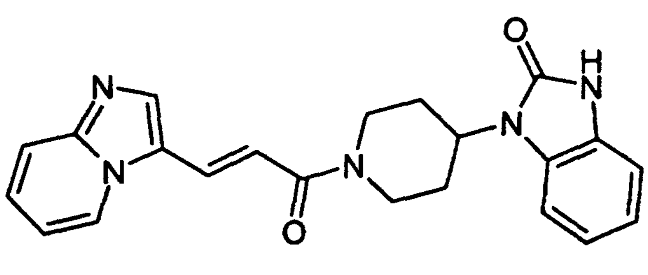

Herstellung analog Beispiel 21; Schmp.: 230°C (Zers.);

**Referenz-Beispiel 24: 1-[3-(6-Chlor-2-methyl-imidazo[1,2-*a*]pyridin-3-yl)-1-oxo-2(*E*)propenyl]-piperidin-4-yl}-1,3-dihydro-benzimidazol-2-on**

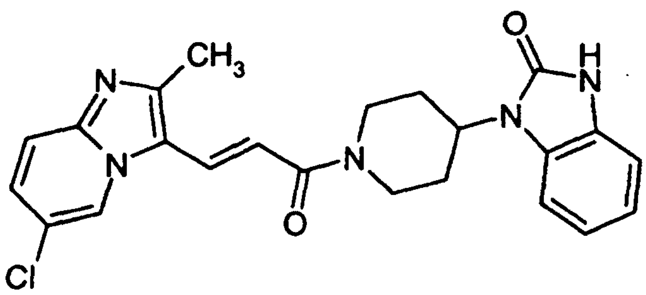

Herstellung analog Beispiel 21; Schmp.: 178-180°C;

**Beispiel 25: 1-{3-[2-(4-Methoxyphenyl)-imidazo[1,2-*a*]pyridin-3-yl]-1-oxo-2(*E*)propenyl}-piperidin-4-yl}-1,3-dihydro-benzimidazol-2-on**

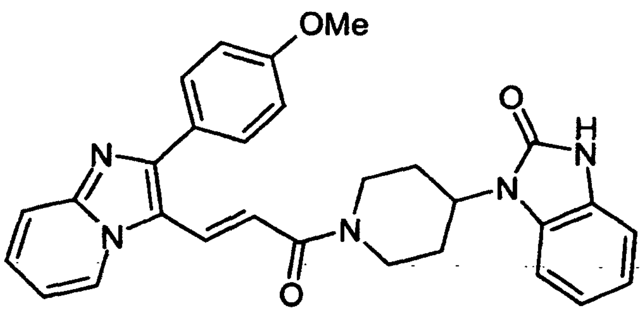

Herstellung analog Beispiel 21; Schmp.: 219°C;

**Beispiel 26: 1-[3-(2-Trofluormethyl-imidazo[1,2-*a*]pyridin-3-yl)-1-oxo-2(*E*)propenyl]-piperidin-4-yl}-1,3-dihydro-benzimidazol-2-on**

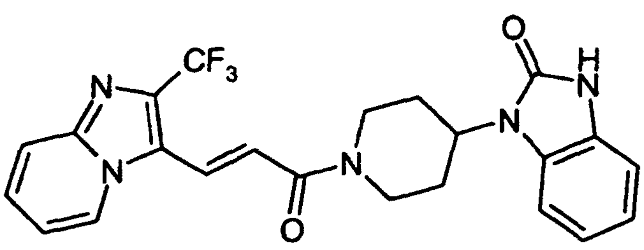

Herstellung analog Beispiel 21; Schmp.: 198°C;

**Referenz-Beispiel 27: 3-{3-[4-(4-Benzyl)-piperidin-1-yl]-1-oxo-2(*E*)-propenyl}-2-phenyl-6-chlor-imidazo[1,2-*a*] pyridin**

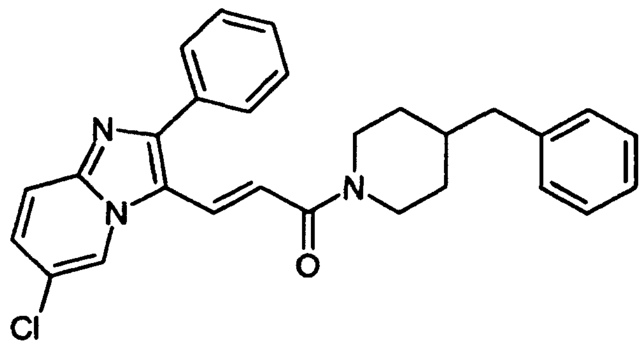

Herstellung analog Beispiel 21; Schmp.: 179-183°C;

**Referenz-Beispiel 28: 3-{3-[4-(4-Phenyl)-piperidin-1-yl]-1-oxo-2(*E*)-propenyl}-2-phenyl-6-chlor-imidazo[1,2-*a*] pyridin**

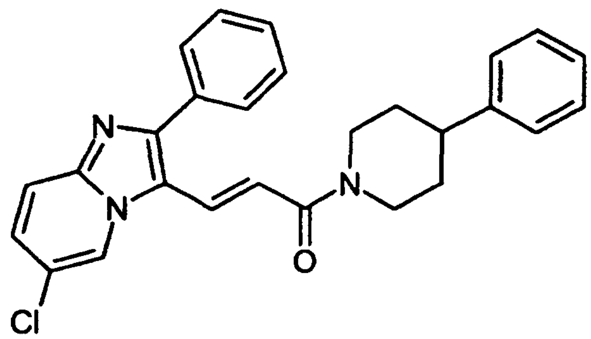

Herstellung analog Beispiel 21; Schmp.: 170-172°C;

Die erfindungsgemäßen Verbindungen zeichnen sich dadurch aus, daß sie den Prozeß der Aß-Entstehung oder Freisetzung aus Zellen inhibieren . Besagte Fähigkeit wurde gemäß der nachfolgenden Testbeschreibung untersucht.

Überprüft wurde die Hemmung der Aß-Bildung im Medium einer APP751 überexprimierenten Zelllinie. Hierzu wurden humane Astroglioma-Zellen (U 373, ATCC) mit dem Gen für das humane APP751 unter Kontrolle eines CMV-Promotors.(Vektor pRC-CMV) stabil transfiziert. Ein Klon mit hoher APP751 Expression (U373-K10) wurde selektioniert. Für die Durchführung des Assays werden die Zellen in 96-Well Platten (Falcon) in Dulbecco's-Minimal-Essential Medium (DMEM, Bio-Whittaker) mit 10 % FCS ausplattiert und bei 37°C / 5 % $CO_2$ bis zur Konfluenz kultiviert.

Die zu testenden Substanzen werden zunächst in 100 % DMSO gelöst und anschließend in Kulturmedium auf Testkonzentration verdünnt, so daß die DMSO Konzentration der Testlösung immer unter 0,5 % liegt.

150 µl der Testlösung werden pro Well auf eine zuvor 1 x mit DMEM gewaschene, konfluente Kulturplatte gegeben und ca. 16 Stunden vorinkubiert. Diese Lösung wird nach 1 x waschen durch 150 µl frische Testlösung ersetzt und erneut für 4 Stunden inkubiert.

100 µl dieses vier Stundenüberstandes werden auf eine, mit dem Antikörper 6E10 (spezifisch für Aβ1-16, Senetek), beschichtete ELISA-Platte (Nunc) übertragen und bei 4°C über Nacht inkubiert. Die Bestimmung von Aβ erfolgt am nächsten Tag mittels des alkalische Phosphatase konjugierten Antikörpers 6B10, der spezifisch für Aβ32-40 ist.

Die sich für die erfindungsgemäßen Verbindungen ergebenden IC50-Werte sind Tabelle 1 zu entnehmen.

Tabelle 1:

| Beispiel | $IC_{50}$-Wert [μM] |
|---|---|
| 1 | 12 |
| 2 | 5 |
| 11 | 6 |
| 12 | 12 |
| 13 | 15 |
| 14 | 30 |
| 20 | 35 |
| 21 | 20 |

Die erfindungsgemäßen Verbindungen können oral, transdermal, intrathecal, inhalativ oder parenteral verabreicht werden und liegen hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragées, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 1 und 5000, vorzugsweise zwischen 10 und 1000, besonders bevorzugt zwischen 10-100 mg/Dosis, bei intravenöser oder intramuskulärer Anwendung zwischen 0,001 und 100, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern bevorzugt. Gleichfalls ist es möglich, die erfindungsgemäßen Verbindungen als Infusionslösung, vorzugsweise in einer physiologischen Kochsalzlösung oder Nährsalzlösung einzusetzen.

Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägem, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt zwischen 1 und 5000 mg, bevorzugt 100 - 1000 mg pro Erwachsener.

Die nachfolgenden Beispiele illustrieren die vorliegende Erfindung ohne sie jedoch in ihrem Umfang zu beschränken:

Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Maisstärke | 190 mg |
| | Milchzucker | 55 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

| C) | Dragées | pro Dragée |
|---|---|---|
| | Wirkstoff | 5 mg |
| | Maisstärke | 41,5 mg |
| | Milchzucker | 30 mg |
| | Polyvinylpyrrolidon | 3 mg |
| | Magnesiumstearat | 0,5 mg |
| | | 80 mg |

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichten aus Zucker und Talkum besteht. Die fertigen Dragees werden mit Wachs poliert.

| D) | Kapseln | pro Kapsel |
|---|---|---|
| | Wirkstoff | 50 mg |
| | Maisstärke | 268,5 mg |

(fortgesetzt)

| D) | Kapseln | pro Kapsel |
|---|---|---|
| | Magnesiumstearat | 1,5 mg<br>320 mg |

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

| E) | Ampullenlösung | |
|---|---|---|
| | Wirkstoff<br>Natriumchlorid<br>Aqua pro inj. | 50 mg<br>50 mg<br>5 ml |

Der Wirkstoff wird bei Eigen-pH oder gegebenenfalls bei pH 5,5 bis 6,5 in Wasser gelöst und mit Natriumchlorid als Isotonans versetzt. die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| F) | Suppositorien | |
|---|---|---|
| | Wirkstoff<br>Adeps solidus | 50 mg<br>1650 mg<br>1700 mg |

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen.

**Patentansprüche**

**1.** Verbindungen der allgemeinen Formel (I)

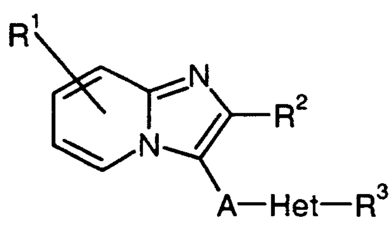

(I)

worin

A $-CH_2-CH_2-$, $-CH_2-CH_2-CH_2-$, $-CH_2-CH_2-CH_2-CH_2-$, $-CH_2-CO$oder $-CH=CH-CO-$ ;
Het Piperidinyl, Piperazinyl oder Dihydrobenzimidazolonyl;
$R^1$ Wasserstoff;
$R^2$ Wasserstoff, $C_1$-$C_4$-Alkyl, $CF_3$ oder ein Phenylrest, der gegebenenfalls durch Halogen, $C_1$-$C_4$-Alkyl oder $C_1$-$C_4$-Alkyloxy substituiert sein kann;
$R^3$ Wasserstoff, $C_1$-$C_4$-Alkyl, HO-$C_1$-$C_4$-Alkyl, $C_2$-$C_4$-Alkenyl, Phenyl oder eine Rest ausgewählt aus der Gruppe bestehend aus Benzyl und Phenylethyl, der gegebenenfalls durch Halogen, $CF_3$, $C_1$-$C_4$-Alkyl oder $C_1$-$C_4$-Alkyloxy substituiert sein kann, oder
ein Heterocyclus ausgewählt aus der Gruppe bestehend aus Morpholin, Piperidin, Piperazin und Dihydrobenzimidazolon, der direkt oder über eine $C_1$-$C_4$-Alkylenbrücke verknüpft sein kann, bedeuten.

**2.** Verbindungen der allgemeinen Formel (I) gemäß Anspruch 1, worin

| | |
|---|---|
| A | $-CH_2-CH_2-$, $-CH_2-CH_2-CH_2-$, $-CH_2-CO-$ oder $-CH=CH-CO-$ ; |
| Het | Piperidinyl, Piperazinyl oder Dihydrobenzimidazolonyl; |
| $R^1$ | Wasserstoff; |
| $R^2$ | Wasserstoff, $C_1$-$C_4$-Alkyl, Phenyl, Halogen-substituiertes Phenyl, $C_1$-$C_4$-Alkyloxy-substituiertes Phenyl oder $CF_3$; |
| $R^3$ | $C_1$-$C_4$-Alkyl, HO-$C_1$-$C_4$-Alkyl, $C_2$-$C_4$-Alkenyl, Phenyl, Benzyl, Phenylethyl, Halogen-substituiertes Benzyl oder ein Heterocyclus ausgewählt aus der Gruppe bestehend aus Morpholin und Dihydrobenzimidazolon, der direkt oder über eine $C_1$-$C_4$-Alkylenbrücke verknüpft sein kann, bedeuten. |

**3.** Verbindungen der allgemeinen Formel (I) gemäß Anspruch 1 oder 2, worin

| | |
|---|---|
| A | $-CH_2-CH_2-$, $-CH_2-CH_2-CH_2-$, $-CH_2-CO-$ oder $-CH=CH-CO-$ ; |
| Het | Piperidinyl, Piperazinyl oder Dihydrobenzimidazolonyl; |
| $R^1$ | Wasserstoff; |
| $R^2$ | Wasserstoff, Methyl, Phenyl oder 4-Chlorphenyl; |
| $R^3$ | $C_1$-$C_4$-Alkyl, HO-$C_1$-$C_4$-Alkyl, $C_2$-$C_4$-Alkenyl, Phenyl, Benzyl, Halogen-substituiertes Benzyl oder ein Heterocyclus ausgewählt aus Morpholin und Dihydrobenzimidazolon, der direkt oder über eine $C_1$-$C_4$-Alkylenbrücke verknüpft sein kann, bedeuten. |

**4.** Verbindungen der allgemeinen Formel (I) gemäß einem der Ansprüche 1 bis 3, worin

| | |
|---|---|
| A | $-CH_2-CH_2-$, $-CH_2-CH_2-CH_2-$ oder $-CH=CH-CO-$ ; |
| Het | Piperidinyl oder Piperazinyl; |
| $R^1$ | Wasserstoff; |
| $R^2$ | Wasserstoff, Methyl, Phenyl oder 4-Chlorphenyl; |
| $R^3$ | 2-Hydroxyethyl, Phenyl, Benzyl, 4-Chlorbenzyl oder 1,3-Dihydrobenzimidazol-2-on-1-yl, bedeuten. |

**5.** Verbindungen der allgemeinen Formel (I) gemäß einem der Ansprüche 1 bis 4, worin

| | |
|---|---|
| A | $-CH_2-CH_2-$, $-CH_2-CH_2-CH_2-$ oder $-CH=CH-CO-$ ; |
| Het | Piperidinyl oder Piperazinyl; |
| $R^1$ | Wasserstoff; |
| $R^2$ | Wasserstoff, Methyl, Phenyl oder 4-Chlorphenyl; |
| $R^3$ | Benzyl, 4-Chlorbenzyl oder 1,3-Dihydrobenzimidazol-2-on-1-yl, |

bedeuten.

**6.** Verbindungen der allgemeinen Formel (I) gemäß einem der Ansprüche 1 bis 5, in denen die Gruppe -Het-$R^3$ für

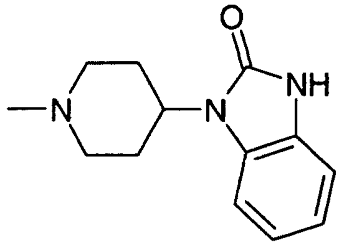

steht.

**7.** Verbindungen der Formel (I) gemäß einem der Ansprüche 1 bis 6 in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren ausgewählt aus der Gruppe der Halogenwasserstoffsäuren oder der organischen Säuren, bevorzugt ausgewählt aus der Gruppe bestehend aus Chlor- oder Bromwasserstoffsäure sowie der Oxal-, Fumar-, Diglycol- oder Methansulfonsäure.

**8.** Arzneimittel **gekennzeichnet durch** einen Gehalt an mindestens einer der Verbindungen der allgemeinen Formel (I) gemäß einem der Ansprüche 1 bis 7.

**9.** Verwendung der Verbindungen der allgemeinen Formel (I) gemäß einem der Ansprüche 1 bis 7 zur Herstellung eines Arzneimittels zur Vorbeugung und/oder Behandlung von Erkrankungen, in denen durch Eingreifen in den Prozeß der Aβ-Entstehung oder Freisetzung aus Zellen durch Inhibition der Aβ-Aktivität ein therapeutischer Nutzen erzielt werden kann.

**10.** Verwendung nach Anspruch 9 zur Herstellung eines Arzneimittels zur Vorbeugung und/oder Behandlung der Alzheimerschen Krankheit.

**11.** Verfahren zur Herstellung von Verbindungen der allgemeinen Formel (I)

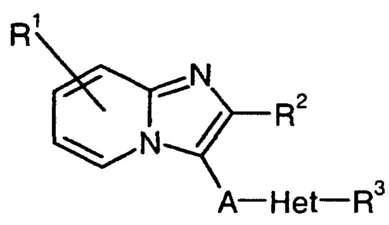

(I)

worin die Reste $R^1$, $R^2$, $R^3$ und Het die in den Ansprüchen 1 bis 7 genannten Bedeutungen haben können und A für $-CH_2-CH_2-$ bzw. $-CH_2-CH_2-CH_2-$ steht, **dadurch gekennzeichnet, daß** eine Verbindung der Formel (VIII)

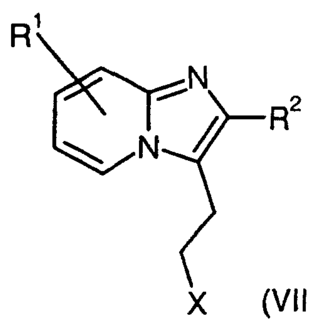

(VIII)

bzw. eine Verbindung der Formel (XVIII)

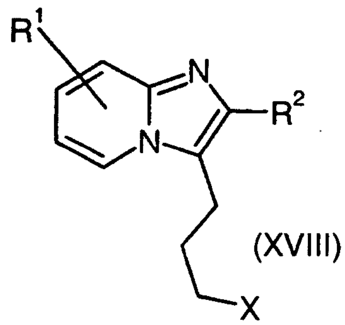

(XVIII)

in denen X für Chlor, Brom oder Iod steht, mit einem Amin der Formel H-Het-$R^3$ umgesetzt wird.

**12.** Verfahren zur Herstellung von Verbindungen der allgemeinen Formel (I)

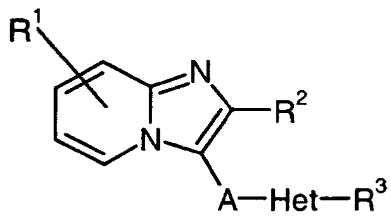

(I)

worin die Reste $R^1$, $R^2$, $R^3$ und Het die in den Ansprüchen 1 bis 7 genannten Bedeutungen haben können und A für $-CH_2-CO-$, $-CH_2-CH_2-CO-$ bzw. - CH=CH-CO- steht, **dadurch gekennzeichnet, daß** eine Carbonsäure der Formel (XIV), (XXII) bzw. (XVI)

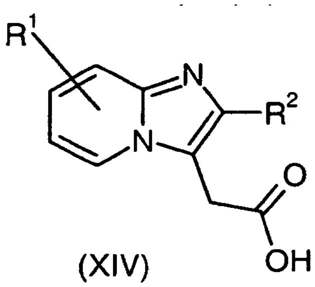

(XIV)

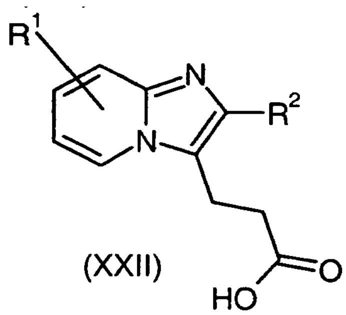

(XXII)

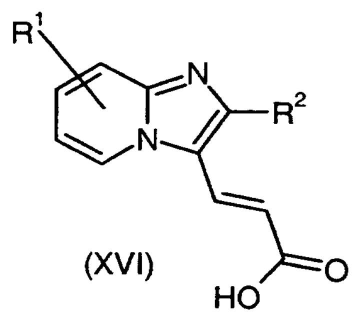

(XVI)

gegebenenfalls unter Verwendung von Kupplungsreagentien mit einem Amin der Formel H-Het-$R^3$ umgesetzt wird.

**13.** Verfahren zur Herstellung von Verbindungen der allgemeinen Formel (I)

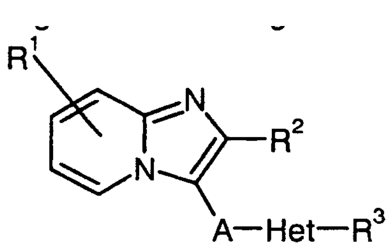

(I)

worin die Reste $R^1$, $R^2$, $R^3$ und Het die in den Ansprüchen 1 bis 7 genannten Bedeutungen haben können und A für $-CH_2-CH_2-$ bzw. $-CH_2-CH_2-CH_2-$ steht, **dadurch gekennzeichnet daß** Verbindungen der Formel (I) in denen A $CH_2-CO-$ bzw. $-CH_2-CH_2-CO-$ bedeuten reduziert werden.

## Claims

**1.** Compounds of general formula (I)

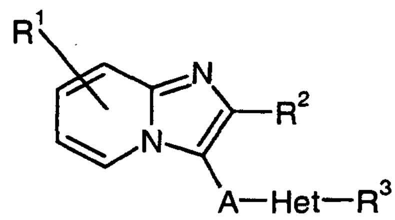

(I)

wherein

A denotes $-CH_2-CH_2-$, $-CH_2-CH_2-CH_2-$, $-CH_2-CH_2-CH_2-CH_2-$, - $CH_2-CO-$ or -CH=CH-CO- ;
Het denotes piperidinyl, piperazinyl or dihydrobenzimidazolonyl;
$R^1$ denotes hydrogen;
$R^2$ denotes hydrogen, $C_1$-$C_4$-alkyl, $CF_3$ or a phenyl group which may optionally be substituted by halogen, $C_1$-$C_4$-alkyl or $C_1$-$C_4$-alkyloxy;
$R^3$ denotes hydrogen, $C_1$-$C_4$-alkyl, HO-$C_1$-$C_4$-alkyl, $C_2$-$C_4$-alkenyl, phenyl or a group selected from among benzyl and phenylethyl, which may optionally be substituted by halogen, $CF_3$, $C_1$-$C_4$-alkyl or $C_1$-$C_4$-alkyloxy, or a heterocycle selected from among morpholine, piperidine, piperazine and dihydrobenzimidazolone, which may be linked directly or via a $C_1$-$C_4$-alkylene bridge.

**2.** Compounds of general formula (I) according to claim 1,
wherein

A denotes $-CH_2-CH_2-$, $-CH_2-CH_2-CH_2-$, $-CH_2-CO-$ or -CH=CH-CO- ;
Het denotes piperidinyl, piperazinyl or dihydrobenzimidazolonyl;
$R^1$ denotes hydrogen;
$R^2$ denotes hydrogen, $C_1$-$C_4$-alkyl, phenyl, halogen-substituted phenyl, $C_1$-$C_4$-alkyloxy-substituted phenyl or $CF_3$;
$R^3$ denotes $C_1$-$C_4$-alkyl, HO-$C_1$-$C_4$-alkyl, $C_2$-$C_4$-alkenyl, phenyl, benzyl, phenylethyl, halogen-substituted benzyl or a heterocycle selected from among morpholine and dihydrobenzimidazolone, which may be linked directly or via a $C_1$-$C_4$-alkylene bridge.

**3.** Compounds of general formula (I) according to claim 1 or 2,
wherein

A denotes $-CH_2-CH_2-$, $-CH_2-CH_2-CH_2-$, $-CH_2-CO-$ or -CH=CH-CO- ;
Het denotes piperidinyl, piperazinyl or dihydrobenzimidazolonyl;
$R^1$ denotes hydrogen;
$R^2$ denotes hydrogen, methyl, phenyl or 4-chlorophenyl;
$R^3$ denotes $C_1$-$C_4$-alkyl, HO-$C_1$-$C_4$-alkyl, $C_2$-$C_4$-alkenyl, phenyl, benzyl, halogen-substituted benzyl or a heterocycle selected from morpholine and dihydrobenzimidazolone, which may be linked directly or via a $C_1$-$C_4$-alkylene bridge.

**4.** Compounds of general formula (I) according to one of claims 1 to 3,
wherein

A denotes $-CH_2-CH_2-$, $-CH_2-CH_2-CH_2-$ or -CH=CH-CO- ;
Het denotes piperidinyl or piperazinyl;
$R^1$ denotes hydrogen;
$R^2$ denotes hydrogen, methyl, phenyl or 4-chlorophenyl;
$R^3$ denotes 2-hydroxyethyl, phenyl, benzyl, 4-chlorobenzyl or 1,3-dihydrobenzimidazol-2-on-1-yl.

**5.** Compounds of general formula (I) according to one of claims 1 to 4, wherein

A denotes $-CH_2-CH_2-$, $-CH_2-CH_2-CH_2-$ or -CH=CH-CO- ;
Het denotes piperidinyl or piperazinyl;
$R^1$ denotes hydrogen;
$R^2$ denotes hydrogen, methyl, phenyl or 4-chlorophenyl;
$R^3$ denotes benzyl, 4-chlorobenzyl or 1,3-dihydrobenzimidazol-2-on-1-yl.

**6.** Compounds of general formula (I) according to one of claims 1 to 5, wherein the group -Het-$R^3$ denotes

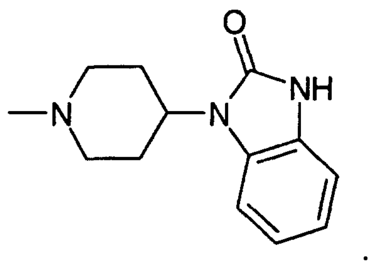

**7.** Compounds of formula (I) according to one of claims 1 to 6 in the form of the individual optical isomers, mixtures of the individual enantiomers or racemates, in the form of the tautomers and in the form of the free bases or the corresponding acid addition salts with pharmacologically acceptable acids selected from among the hydrohalic acids or the organic acids, preferably selected from among hydrochloric or hydrobromic acid and oxalic, fumaric, diglycolic or methanesulphonic acid.

**8.** Pharmaceutical composition, **characterised in that** it contains at least one of the compounds of general formula (I) according to one of claims 1 to 7.

**9.** Use of the compounds of general formula (I) according to one of the claims 1 to 7 for preparing a pharmaceutical composition for the prevention and/or treatment of diseases in which a therapeutic benefit can be obtained by interfering in the process of the formation of Aβ or its release from cells, by inhibiting the Aβ activity.

**10.** Use according to claim 9 for preparing a pharmaceutical composition for the prevention and/or treatment of Alzheimer's disease.

**11.** Process for preparing compounds of general formula (I)

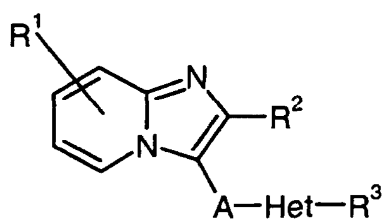
(I)

wherein the groups $R^1$, $R^2$, $R^3$ and Het may have the meanings given in claims 1 to 7 and A denotes $-CH_2-CH_2-$ or $-CH_2-CH_2-CH_2-$, **characterised in that** a compound of formula (VIII)

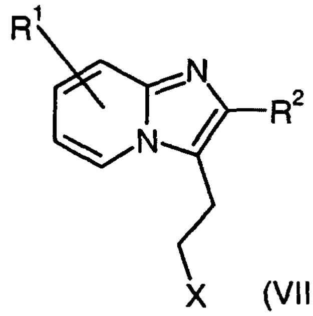

(VIII)

or a compound of formula (XVIII)

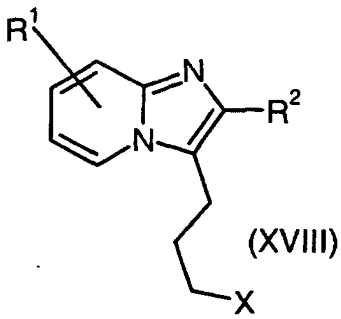

(XVIII)

wherein X denotes chlorine, bromine or iodine, is reacted with an amine of formula H-Het-$R^3$.

**12.** Process for preparing compounds of general formula (I)

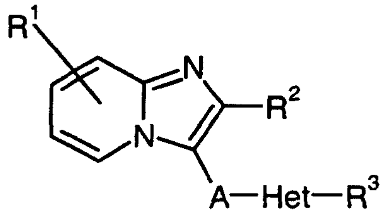

(I)

wherein the groups $R^1$, $R^2$, $R^3$ and Het may have the meanings given in claims 1 to 7 and A denotes $-CH_2-CO-$, $-CH_2-CH_2-CO-$ or $-CH=CH-CO-$, **characterised in that** a carboxylic acid of formula (XIV), (XXII) or (XVI)

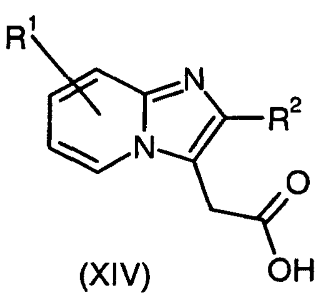

(XIV)

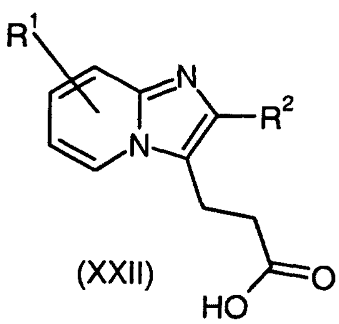

(XXII)

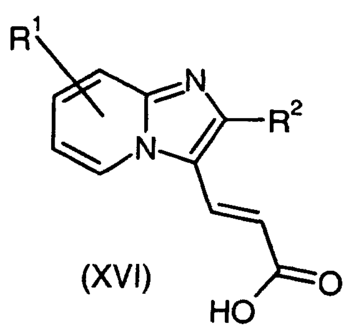

(XVI)

is reacted with an amine of formula H-Het-$R^3$ , optionally using coupling reagents.

**13.** Process for preparing compounds of general formula (I)

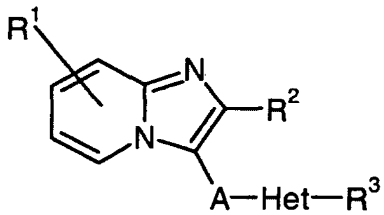

(I)

wherein the groups $R^1$, $R^2$, $R^3$ and Het may have the meanings given in claims 1 to 7 and A denotes $-CH_2-CH_2-$ or $-CH_2-CH_2-CH_2-$, **characterised in that** compounds of formula (I) wherein A denotes $CH_2$-CO- or $-CH_2-CH_2$-CO- are reduced.

## Revendications

**1.** Composés de formule générale (I)

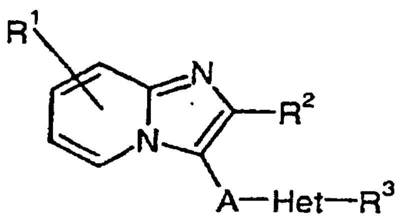

(I)

où
A représente $-CH_2-CH_2-$, $-CH_2-CH_2-CH_2-$,$-CH_2-CH_2-CH_2-CH_2-$,$-CH_2$-CO- ou - CH=CH-CO-;
Het représente pipéridinyle, pipérazinyle ou dihydrobenzimidazolonyle;
$R^1$ représente l'hydrogène ;
$R^2$ représente l'hydrogène, $C_1$-$C_4$-alkyle, $CF_3$ ou un groupement phényle, qui peut éventuellement être substitué par halogène, $C_1$-$C_4$-alkyle ou $C_1$-$C_4$-alkyloxy ;
$R^3$ représente l'hydrogène, $C_1$-$C_4$-alkyle, HO-$C_1$-$C_4$-alkyle, $C_2$-$C_4$-alcényle, phényle ou un groupement choisi dans le groupe consistant en benzyle et phényléthyle, qui peut éventuellement être substitué par halogène, $CF_3$, $C_1$-$C_4$-alkyle ou $C_1$-$C_4$-alkyloxy, ou
un hétérocycle choisi dans le groupe consistant en la morpholine, la pipéridine, la pipérazine et la dihydrobenzimidazolone, qui peut être lié directement ou par le biais d'un pont $C_1$-$C_4$-alkylène.

**2.** Composés de formule générale (I) selon la revendication 1
où
A représente $-CH_2-CH_2-$, $-CH_2-CH_2-CH_2-$,$-CH_2$-CO- ou -CH=CH-CO-;
Het représente pipéridinyle, pipérazinyle ou dihydrobenzimidazolonyle;
$R^1$ représente l'hydrogène ;
$R^2$ représente l'hydrogène, $C_1$-$C_4$-alkyle, phényle, phényle halogéno-substitué, phényle $C_1$-$C_4$-alkyloxy-substitué ou $CF_3$ ;
$R^3$ représente $C_1$-$C_4$-alkyle, HO-$C_1$-$C_4$-alkyle, $C_2$-$C_4$-alcényle, phényle, benzyle, phényléthyle, benzyle halogéno-substitué ou un hétérocycle choisi dans le groupe consistant en la morpholine et la dihydrobenzimidazolone, qui peut être lié directement ou par le biais d'un pont $C_1$-$C_4$-alkylène.

3. Composés de formule générale (I) selon la revendication 1 ou 2 où
A représente $-CH_2-CH_2-$, $-CH_2-CH_2-CH_2-$,$-CH_2-CO-$ ou $-CH=CH-CO-$;
Het représente pipéridinyle, pipérazinyle ou dihydrobenzimidazolonyle;
$R^1$ représente l'hydrogène ;
$R^2$ représente l'hydrogène, méthyle, phényle ou 4-chlorophényle ;
$R^3$ représente $C_1$-$C_4$-alkyle, HO-$C_1$-$C_4$-alkyle, $C_2$-$C_4$-alcényle, phényle, benzyle, benzyle halogéno-substitué ou un hétérocycle choisi parmi la morpholine et la dihydrobenzimidazolone, qui peut être lié directement ou par le biais d'un pont $C_1$-$C_4$-alkylène.

4. Composés de formule générale (I) selon l'une des revendications 1 à 3 où
A représente $-CH_2-CH_2-$, $-CH_2-CH_2-CH_2-$ ou $-CH=CH-CO-$;
Het représente pipéridinyle ou pipérazinyle;
$R^1$ représente l'hydrogène ;
$R^2$ représente l'hydrogène, méthyle, phényle ou 4-chlorophényle ;
$R^3$ représente 2-hydroxyéthyle, phényle, benzyle, 4-chlorobenzyle ou 1,3-dihydrobenzimidazol-2-on-1-yle.

5. Composés de formule générale (I) selon l'une des revendications 1 à4 où
A représente $-CH_2-CH_2-$, $-CH_2-CH_2-CH_2-$ ou $-CH=CH-CO-$;
Het représente pipéridinyle ou pipérazinyle;
$R^1$ représente l'hydrogène ;
$R^2$ représente l'hydrogène, méthyle, phényle ou 4-chlorophényle ;
$R^3$ représente benzyle, 4-chlorobenzyle ou 1,3-dihydrobenzimidazol-2-on-1-yle.

6. Composés de formule générale (I) selon l'une des revendications 1 à 5 dans lesquels le groupe -Het-$R^3$ représente

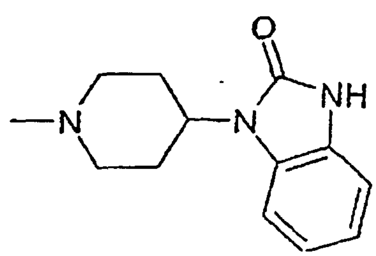

7. Composés de formule (I) selon l'une des revendications 1 à 6 sous forme des différents isomères optiques, de mélanges des différents énantiomères ou de racémates, sous forme des tautomères ainsi que sous forme des bases libres ou des sels d'addition d'acide correspondants avec des acides pharmacologiquement acceptables choisis dans le groupe des acides halogénhydriques ou des acides organiques, de préférence choisis dans le groupe consistant en l'acide chlor- ou bromhydrique ainsi que l'acide oxalique, fumarique, diglycolique ou méthanesulfonique.

8. Médicament **caractérisé par** une teneur en au moins l'un des composés de formule générale (I) selon l'une des revendications 1 à 7.

9. Utilisation des composés de formule générale (I) selon l'une des revendications 1 à 7 pour la préparation d'un médicament pour la prévention et/ou le traitement de maladies dans lesquelles un profit thérapeutique peut être obtenu par intervention dans le processus de production ou de libération de Aβ à partir de cellules par inhibition de l'activité de Aβ.

10. Utilisation selon la revendication 9 pour la production d'un médicament pour la prévention et/ou le traitement de la maladie d'Alzheimer.

**11.** Procédé de préparation de composés de formule générale (I)

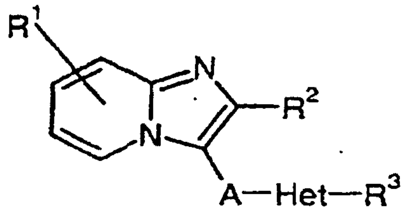

(I)

où les groupements $R^1$, $R^2$, $R^3$ et Het peuvent avoir les significations citées dans les revendications 1 à 7 et A représente $-CH_2-CH_2-$ ou $-CH_2-CH_2-CH_2-$, **caractérisé en ce qu'**un composé de formule (VIII)

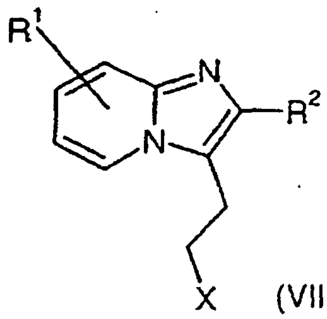

(VIII)

ou un composé de formule (XVIII)

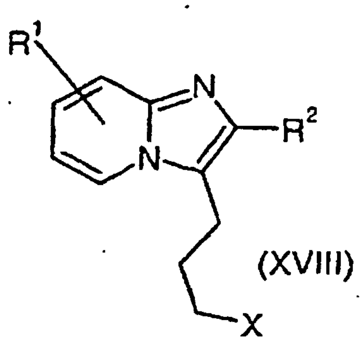

(XVIII)

dans lesquels X représente le chlore, le brome ou l'iode, est mis à réagir avec une amine de formule $H\text{-}Het\text{-}R^3$.

**12.** Procédé de préparation de composés de formule générale (I)

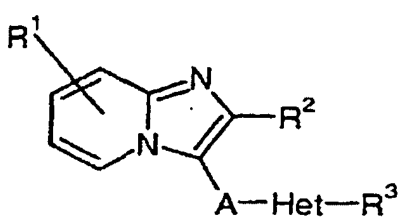

(I)

où les groupements $R^1$, $R^2$, $R^3$ et Het peuvent avoir les significations citées dans les revendications 1 à 7 et A représente $-CH_2-CO-$, $-CH_2-CH_2-CO-$ ou - CH=CH-CO-, **caractérisé en ce qu'**un acide carboxylique de formule (XIV), (XXII) ou (XVI)

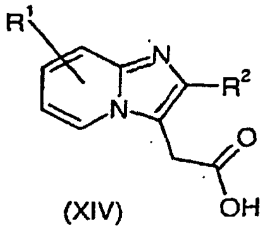
(XIV)
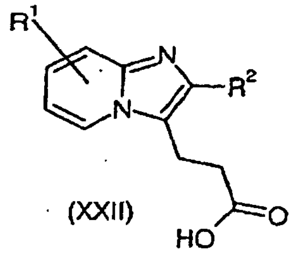
(XXII)
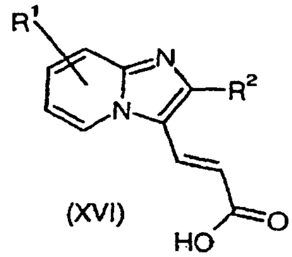
(XVI)

est mis à réagir avec une amine de formule H-Het-$R^3$ éventuellement au moyen de réactifs de couplage.

**13.** Procédé de préparation de composés de formule générale (I)

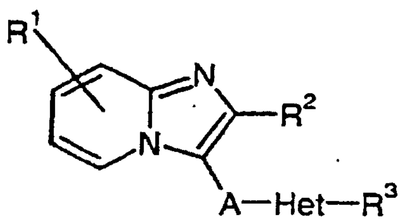
(I)

où les groupements $R^1$, $R^2$, $R^3$ et Het peuvent avoir les significations citées dans les revendications 1 à 7 et A représente $-CH_2-CH_2-$ ou $--CH_2-CH_2-CH_2-$, **caractérisé en ce que** des composés de formule (I) dans lesquels A représente $-CH_2-CO-$ ou $-CH_2-CH_2-CO-$ sont réduits.